# EUROPEAN PATENT SPECIFICATION

(11) **EP 0 540 609 B1**
(45) Date of publication and mention of the grant of the patent: **13.10.1999**
(21) Application number: 91913583.0
(22) Date of filing: 22.07.1991
(51) Int. Cl.: C07D 501/20, C07D 501/18, C07D 463/00, A61K 31/545, A61K 31/435

(54) **CEPHALOSPORINS AND HOMOLOGUES, PREPARATIONS AND PHARMACEUTICAL COMPOSITIONS**
CEPHALOSPORINE UND IHRE HOMOLOGEN, ZUBEREITUNGEN UND PHARMAZEUTSICHE PRÄPARATE
CEPHALOSPORINES ET HOMOLOGUES, PREPARATIONS ET COMPOSITIONS PHARMACEUTIQUES

(30) Priority: 24.07.1990 GB 9016189; 02.05.1991 GB 9109540
(43) Date of publication of application: 12.05.1993
(73) Proprietor: PFIZER INC., New York, N.Y. 10017-5755 (US)
(72) Inventor: BATESON, John, Hargreaves SmithKline Beecham, Betchworth Surrey RH3 7AJ (GB); BURTON, George SmithKline Beecham Pharmaceuticals, Betchworth Surrey RH3 7AJ (GB); FELL, Stephen, Christopher, Martin, Betchworth Surrey RH3 7AJ (GB)
(74) Representative: Wood, David John
(86) International application number: GB9101228
(87) International publication number: WO9201696

(56) References cited:
- EP-A- 0 395 219
- FR-A- 2 183 892
- FR-A- 2 197 573
- US-A- 3 962 223
- KOICHI FUJIMOTO et al.:"Studies on Orally active Cephalosporin Esters", THE JOURNAL OF ANTIBIOTICS, 01-03-1987, vol. 40, no. 3, pages 370 to 384
- DRUGS OF THE FUTURE, vol. 14, no. 1, pages 73 to 74
- JOHN H. BATESON et al.: "Novel C-3 Cyclic Ethar Cephalosporins and their orally absorbed Prodrug Esters", THE JOURNAL OF ANTIBIOTICS, 01-02-1994, vol. 47, no. 2, pages 253 to 256

## Description

This invention relates to novel β-lactam containing compounds, their preparation and their use, and in particular to a novel class of cephalosporins. These compounds have antibacterial properties, and are therefore of use in the treatment of bacterial infections in humans and animals caused by a wide range of organisms.

GB 1 385 831 (Hoechst) claims 7-acylamino-cephem-carboxylic acid compounds substituted at the 7-position by a group: 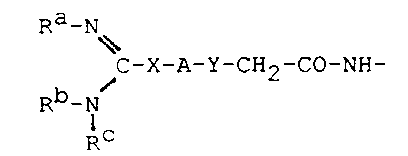 in which R^{a} and R^{b}, which may be the same or different, each represents a hydrogen atom or an alkyl group having from 1 to 5 carbon atoms or R^{a} and R^{b} together represent an alkylene group which may be substituted, R^{c} represents a hydrogen atom or an alkyl group having from 1 to 5 carbon atoms, X represents a single bond or an NH group, A represents a phenylene or thienylene group which may be substituted and Y represents a single bond or an oxygen atom;
and substituted at the 3-position by an alkyl group having from 1 to 5 carbon atoms, or a cyclo-alkyl group having from 3 to 7 ring carbon atoms which may include one or more hetero ring atoms. Tetrahydrofuranyl is described as an example of a 3-position substituent from a list of 14 radicals. The Examples describe only methyl, ethyl and isopropyl groups at the 3-position of the cephalosporin nucleus.

Cephalosporins bearing an ether substituent at the 3-position of the cephalosporin nucleus are known from "Studies or Orally Active Cephalosporin Esters", by K. Fujimoto et al, J. Antibiotics (March 1987), pages 370 to 384 and Drugs of the Future, Vol. 14, No 1, 1989.

We have now found a particular class of cephalosporins bearing a cyclic ether substituent at the 3-position of the cephalosporin nucleus that possesses prolonged and high levels of antibacterial activity, and shows good absorption both parentally and orally, especially orally.

The present invention provides a compound of formula (I) or a salt thereof 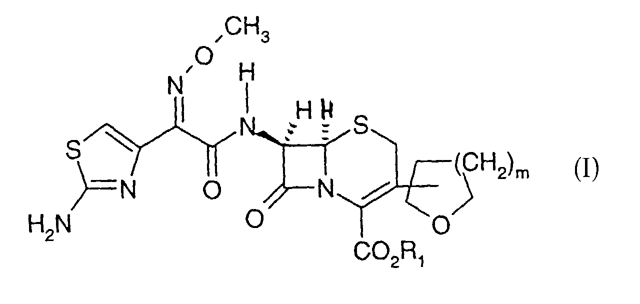 wherein m is 1 or 2,
and CO₂R₁ is a carboxy group or a carboxylate anion, or R¹ is a readily removable carboxy protecting group.

The bonding carbon atom of the cyclic ether moiety which links the ring to the cephalosporin nucleus is generally asymmetric. The present invention includes either stereoisomer, as well as mixtures of both isomers.

Since the β-lactam antibiotic compounds of-the present invention are intended for use as therapeutic agents in pharmaceutical compositions, it will be readily appreciated that preferred compounds within formula (I) are pharmaceutically acceptable, i.e. are compounds of formula (Ia) 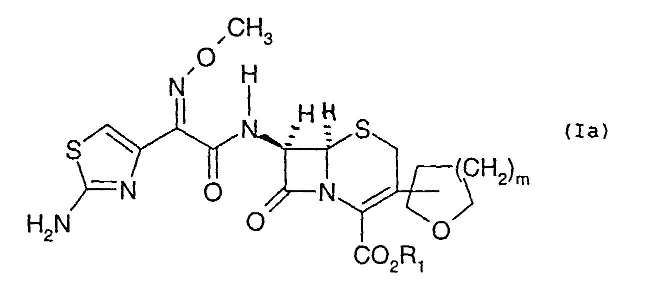 wherein the group CO₂R₁ is a carboxy group or a carboxylate anion, or a pharmaceutically acceptable salt or in vivo hydrolysable ester thereof.

Accordingly, the present invention provides a compound of formula (Ia) or a pharmaceutically acceptable salt or in vivo hydrolysable ester thereof, for use as a therapeutic agent, and in particular an in vivo hydrolysable ester thereof for use as an orally administrable therapeutic agent.

The present invention further provides a compound of formula (Ia) or a pharmaceutically acceptable salt or in vivo hydrolysable ester thereof, for use in the treatment of bacterial infections, more particularly an in vivo hydrolysable ester thereof for use in the oral treatment of bacterial infections.

The present invention also includes a method of treating bacterial infections in humans and animals which comprises the administration of a therapeutically effective amount of an antibiotic compound of this invention of the formula (Ia) or a pharmaceutically acceptable in vivo hydrolysable ester thereof, in particular the oral administration of a therapeutically effective amount of an in vivo hydrolysable ester.

In addition, the present invention includes the use of a compound of formula (Ia) or a pharmaceutically acceptable salt or in vivo hydrolysable ester thereof, for the manufacture of a medicament for the treatment of bacterial infections, in particular the use of an in vivo hydrolysable ester for the manufacture of a medicament for the oral treatment of bacterial infections.

Those compounds of the formula (I) wherein R¹ is a readily removable carboxy protecting group other than a pharmaceutically acceptable in vivo hydrolysable ester or which are in non-pharmaceutically acceptable salt form are primarily useful as intermediates in the preparation of compounds of the formula (Ia) or a pharmaceutically acceptable salt or pharmaceutically acceptable in vivo hydrolysable ester thereof.

Suitable readily removable carboxy protecting groups for the group R¹ include groups forming ester derivatives of the carboxylic acid, including in vivo hydrolysable esters. The derivative is preferably one which may readily be cleaved in vivo.

It will be appreciated that also included within the scope of the invention are salts and carboxy-protected derivatives, including in vivo hydrolysable esters, of any carboxy groups that may be present as optional substituents in compounds of formula (I) or (Ia). Also included within the scope of the invention are acid addition salts of the amino group that is be present as optional substituents in compounds of formula (I) or (Ia).

Suitable ester-forming carboxyl-protecting groups are those which may be removed under conventional conditions. Such groups for R¹ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus- containing group, an oxime radical of formula -N=CHR⁷ where R⁷ is aryl or heterocyclic, or an in vivo hydrolysable ester radical such as defined below.

When used herein the term 'aryl' includes phenyl and naphthyl, each optionally substituted with up to five, preferably up to three, groups selected from halogen, mercapto, C₁₋₆ alkyl, phenyl, C₁₋₆ alkoxy, hydroxy(C₁₋₆)alkyl, mercapto(C₁₋₆)alkyl, halo(C₁₋₆) alkyl, hydroxy, amino, nitro, carboxy, C₁₋₆ alkylcarbonyloxy, alkoxycarbonyl, formyl, or C₁₋₆ alkylcarbonyl groups.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected from halogen, (C₁₋₆)alkyl, (C₁₋₆)alkoxy, halo(C₁₋₆)alkyl, hydroxy, carboxy, carboxy salts, carboxy esters such as (C₁₋₆)alkoxycarbonyl, (C₁₋₆)alkoxycarbonyl(C₁-C₆)alkyl, aryl, and oxo groups. Each heterocyclic ring suitably has from 4 to 7, preferably 5 or 6, ring atoms. The term 'heteroaryl' refers to heteroaromatic heterocyclic rings. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

When used herein the terms 'alkyl' alkenyl, alkynyl and 'alkoxy' include straight and branched chain groups containing from 1 to 6 carbon atoms, such as methyl, ethyl, propyl and butyl. A particular alkyl group is methyl.

When used herein the term 'halogen' refers to fluorine, chlorine, bromine and iodine.

A carboxyl group may be regenerated from any of the above esters by usual methods appropriate to the particular R¹ group, for example, acid- and base- catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formulae (i), (ii), (iii), (iv) and (v):

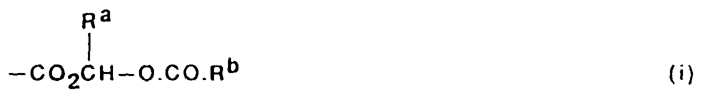

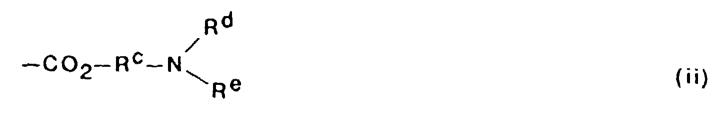

-CO₂CH₂-OR^{f} (iii)

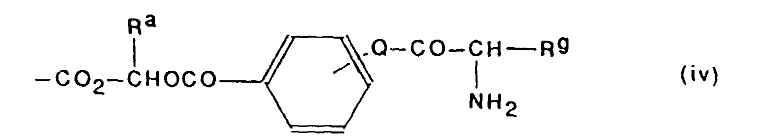

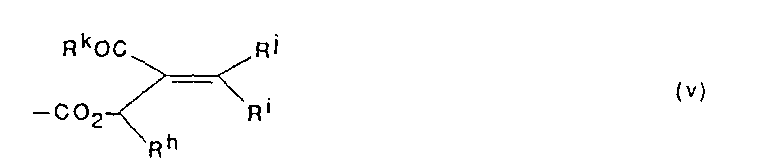

wherein R^{a} is hydrogen, C₁₋₆ alkyl, C₃₋₇ cycloalkyl, methyl, or phenyl, R^{b} is C₁₋₆ alkyl, C₁₋₆ alkoxy, phenyl, benzyl, C₃₋₇ cycloalkyl, C₃₋₇ cycloalkyloxy, C₁₋₆ alkyl C₃₋₇ cycloalkyl, 1-amino C₁₋₆ alkyl, or 1-(C₁₋₆ alkyl)amino C₁₋₆ alkyl; or R^{a} and R^{b} together form a 1,2-phenylene group optionally substituted by one or two methoxy groups; R^{c} represents C₁₋₆ alkylene optionally substituted with a methyl or ethyl group and R^{d} and R^{e} independently represent C₁₋₆ alkyl; R^{f} represents C₁₋₆ alkyl; R^{g} represents hydrogen or phenyl optionally substituted by up to three groups selected from halogen, C₁₋₆ alkyl, or C₁₋₆ alkoxy; Q is oxygen or NH; R^{h} is hydrogen or C₁₋₆ alkyl; Rⁱ is hydrogen, C₁₋₆ alkyl optionally substituted by halogen, C₂₋₆ alkenyl, C₁₋₆ alkoxycarbonyl, aryl or heteroaryl; or R^{h} and Rⁱ together form C₁₋₆ alkylene; R^{j} represents hydrogen, C₁₋₆ alkyl or C₁₋₆ alkoxycarbonyl; and R^{k} represents C₁₋₈ alkyl, C₁₋₈ alkoxy, C₁₋₆ alkoxy(C₁₋₆)alkoxy or aryl.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl, α-ethoxycarbonyloxyethyl and propoxycarbonyloxyethyl; dialkylaminoalkyl especially di-loweralkylamino alkyl groups such as dimethylaminomethyl, dimethylaminoethyl, diethylaminomethyl or diethylaminoethyl; 2-(alkoxycarbonyl)-2-alkenyl groups such as 2-(isobutoxycarbonyl)pent-2-enyl and 2-(ethoxycarbonyl)but-2-enyl; lactone groups such as phthalidyl and dimethoxyphthalidyl; and esters linked to a second β-lactam antibiotic or to a β-lactamase inhibitor.

A preferred in vivo hydrolysable ester group is the pivaloyloxymethyl ester.

A further suitable pharmaceutically acceptable in vivo hydrolysable ester group is that of the formula: 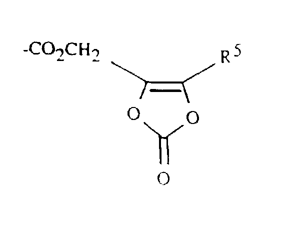 wherein R⁵ is hydrogen, C₁₋₆ alkyl or phenyl.

Suitable pharmaceutically acceptable salts of the carboxy group of the compound of formula (I) include metal salts, eg aluminium, alkali metal salts such as sodium or potassium, especially sodium, alkaline earth metal salts such as calcium or magnesium, and ammonium or substituted ammonium salts, for example those with lower alkylamines such as triethylamine, hydroxy-lower alkylamines such as 2-hydroxyethylamine, bis- (2-hydroxyethyl) amine or tris-(2-hydroxyethyl)- amine, cycloalkylamines such as dicyclohexylamine, or with procaine, dibenzylamine, N,N-dibenzylethylene- diamine, 1-ephenamine, N-methylmorpholine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other useful salts include the lithium salt and silver salt. Salts within compounds of formula (I), may be prepared by salt exchange in conventional manner.

Preferably m is 1.

Preferably the cyclic ether is bonded to the cephalosporin nucleus at a ring carbon adjacent to the oxygen heteroatom.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the antibiotic compounds of the invention are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 49% of a compound of the formula (I) or salt thereof.

Specific compounds within this invention of formula (Ia) include the following pharmaceutically acceptable carboxylic acids, salts and in-vivo hydrolysable esters:
Sodium (6R,7R)-7-[2-(2-Aminothiazol-4-yl)-2-(Z)-methoxyiminoacetamido]-3-[(RS)-tetrahydrofuran-2-yl]ceph-3-em-4-carboxylate;
Pivaloyloxymethyl (6R,7R)-7-[2-(2-Aminothiazol-4-yl)-2-(Z)-methoxyiminoacetamido]-3-[(RS)-tetrahydrofuran-2-yl]ceph-3-em-4-carboxylate;
Sodium (6R,7R-7-[2-(2-Aminothiazol-4-yl)-2-(Z)-methoxyirninoacetamido]-3-[(RS)-tetrahydropyran-2-yl]ceph-3-em-4-carboxylate;
Sodium (6R,7R)-7-[2-(2-aminothiazol-4-yl)-2-(Z)-methixyiminoacetamido]-3-[(S)-tetrahydrofuran-2-yl]ceph-3-em-4-carboxylate;
Pivaloyloxymethyl (6R,7R)-7-[2-(2-aminothiazol-4-yl)-2-(Z)-methoxyiminoacetamido]-3-[(S)-tetrahydrofuran-2-yl]ceph-3-em-4-carboxylate;
Sodium (6R,7R)-7-[2-(2-Aminothiazol-4-yl)-2-(Z)-methoxyiminoacetarnido]-3-[(R)-tetrahydrofuran-2-yl]ceph-3-em-4-carboxylate;
Pivaloyloxymethyl (6R,7R)-7-[2-(2-aminothiazol-4-yl)-2-(Z)-methoxyiminoacetamido]-3-[(R)-tetrahydrofuran-2-yl]ceph-3-em-4-carboxylate;
Sodium (6R,7R)-7-[2-(2-Aminothiazol-4-yl)-2-(Z)-methoxyiminoacetamido-3-[(RS)-tetrahydrofuran-3-yl]ceph-3-em-4-carboxylate;
Acetoxymethyl (6R,7R)-7-[2-(2-aminothiazol-4-yl)-2-(Z)-methoxyiminoacetamido]-3-[-tetrahydrofuran-2-yl]-ceph-3-em-4-carboxylate;
(RS)-1-Acetoxyethyl (6R,7R)-7-[2-(2-Aminothiazol-4-yl)-2-(Z)-methoxyiminoacetamido]acetamido]-3-[(S)-tetrahydrofuran-2-yl]ceph-3-em-4-carboxylate;
(RS)-1 -(Propan-2-yl)oxycarbonyloxyethyl (6R,7R)-7-[2-(2-aminothiazol-4-yl)-2-(Z)-methoxyiminoacetamido]-3-[(S)-tetrahydrofuran-2-yl]ceph-3-em-4-carboxylate; and
2-Ethoxycarbonyl-(Z)-but-2-enyl (6R,7R]-7-[2-(2-aminothiazol-4-yl)-2-(Z)-methoxyiminoacetamido[-3-[(S)-tetrahydrofuran-2-yl]ceph-3-em-4-carboxylate.

The present invention further provides a process for the preparation of a compound of formula (I), which process comprises treating a compound of formula (II) or a salt thereof:
(a) treating a compound of formula (II) or a salt thereof 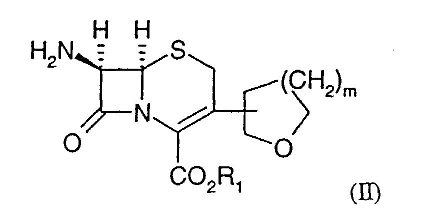 wherein CO₂R₁ and m are as defined in claim 1 or 2, and wherein any reactive group may be protected, and wherein the amino group is optionally substituted with a group which permits acylation to take place, with an N-acylating derivative of an acid of formula (III):

   R²-OH (III)

   where R² = 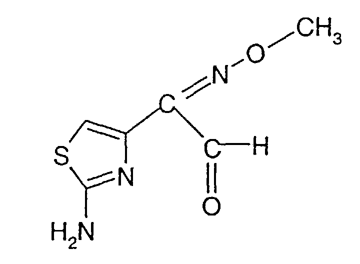 wherein any reactive group may be protected; or
(b) cyclising a compound of formula (IV): 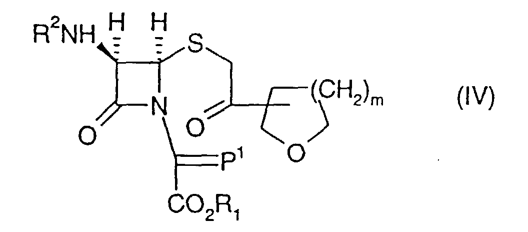 wherein R², m and CO₂R₁ are as defined in (a) above and P is a phosphorus residue; or
(c) treating a compound of formula (X): 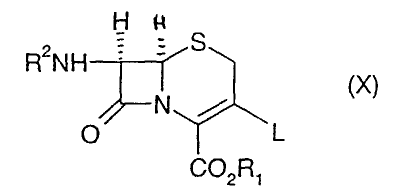 wherein R² and CO₂R₁ are as defined in (a) above and L is a leaving group, with a compound of formula (Xl): 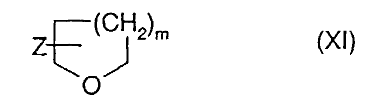 wherein Z is an organo-cuprate group and m is as defined in (a) above;
and thereafter, if necessary or desired, carrying out one of the following steps:
i) removing any protecting groups;
ii) converting the group CO₂R₁ to a different group CO₂R₁;
iii) converting the product to a salt.

Acids of formula (III) may be prepared by methods known in the art, or methods analogous to such processes. Suitable o processes include those described, for example, in UK Patent 2 107 307 B, UK Patent Specification No. 1,536,281, and U.K. Patent Specification No. 1,508,064.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-n-butyltin, groups of formula -P.R²⁰R²¹ wherein R²⁰ is an alkyl, haloalkyl, aryl, aralkyl, alkoxy, haloalkyl, aryl, aralkyl, alkoxy, haloalkoxy, aryloxy, aralkyloxy or dialkylamino group, R²¹ is the same as R²⁰ or is halogen or R²⁰ and R²¹ together form a ring; suitable such phosphorus groups being -P(OC₂H₅)₂, -P(C₂H₅)₂,

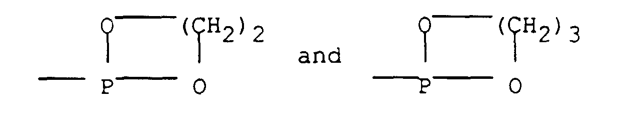

A group which may optionally be introduced onto the amino group in the compound of formula (II) is trimethylsilyl.

Advantageously the silylation reaction may be carried out in situ, prior to the acylation reaction, with a silylating agent that does not require concomitant addition of base. Suitable silylating agents include, for example, N-(trimethylsilyl)-acetamide, N,O-bis-(trimethylsilyl)acetamide, N,O-bis(trimethylsilyl)trifluoroacetamide, N-methyl-N-trimethylsilylacetamide, N-methyl-N-trimethylsilyl-trifluoroacetamide, N,N'-bis(trimethylsilyl)urea, and N,O-bis(trimethylsilyl)carbamate. A preferred silylating agent is N,O-bis(trimethylsilyl)acetamide. The silylation reaction may suitably be carried out in an inert, anhydrous organic solvent such as dichloromethane at room temperature or at an elevated temperature, for example 30 - 60°C, preferably 40 - 50°C.

The above process may optionally be carried out in the presence of a small quantity, for example 0.1 equivalents, of a silyl halide, for example a tri(C₁₋₆)alkylsilyl halide, especially trimethylsilyl chloride.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide or alternatively a symmetrical or mixed anhydride. The acylation may be effected in the presence of an acid binding agent for example, tertiary amine (such as pyridine or dimethylaniline), molecular sieves, an inorganic base (such as calcium carbonate or sodium bicarbonate) or an oxirane, which binds hydrogen halide liberated in the acylation reaction. The oxirane is preferably a (C₁₋₆)-1,2-alkylene oxide - such as ethylene oxide or propylene oxide. The acylation reaction using an acid halide may be carried out at a temperature in the range -50°C to +50°C, preferably -20°C to +20°c, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate. The acylation with acid halide or anhydride is suitably carried out in the presence of a basic catalyst such as pyridine or 2,6-lutidine.

Acid halides may be prepared by reacting the acid (III) or a salt or a reactive derivative thereof with a halogenating (eg chlorinating or brominating) agent such as phosphorus pentachloride, thionyl chloride, oxalyl chloride or phosgene.

Suitable mixed anhydrides are anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid).

Alternative N-acylating derivatives of acid (III) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (III) with an oxime.

Other reactive N-acylating derivatives of the acid (III) include the reactive intermediates formed by reaction in situ with a condensing agent such as a carbodiimide, for example, N,N'-diethyl-, dipropyl- or diisopropylcarbodiimide, N,N'-di-cyclohexyl-carbodiimide, or N-ethyl-N'-[3-(dimethylamino)propyl]- carbodiimide; a suitable carbonyl compound, for example, N,N'-carbonyldiimidazole or N,N'-carbonyldi- triazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example BBr₃ - C₆H₆); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

A further method of forming the N-acylating derivative of the acid of formula (III) is to treat the acid of formula (III) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid of formula (III) so derived may then be caused to react with a compound of formula (II). The acylation reaction may conveniently be carried out at -40° to +30°C, if desired in the presence of an acid binding agent such as pyridine. A catalyst such as 4-dimethylaminopyridine may optionally also be added. A preferred solvent for the above acylation reaction is dichloromethane.

In the process described hereinabove, and in-the process described hereinbelow, it may be necessary to remove protecting groups. Deprotection may be carried out by any convenient method knqwn in the art such that unwanted side reactions are minimised. Separation of unwanted by-products may be carried out using standard methods.

The cyclisation reaction is an intramolecular Wittig-type reaction and is typically carried out by heating the compound of formula (IV) in an organic solvent system, for example in toluene, optionally in the presence of a suitable acid such as benzoic acid.

A compound of formula (IV) may be prepared from a compound of formula (V): 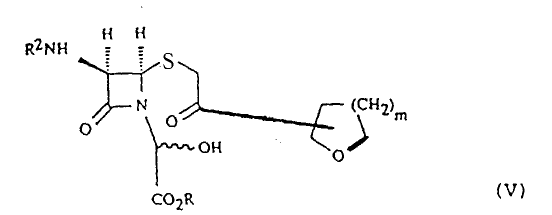 wherein R², m, and CO₂R¹ are as hereinbefore defined, by reaction with a halogenating agent, suitably a chlorinating agent such as thionyl chloride, which reaction displaces the formula (V) hydroxyl group by halogen, suitably chloride, and is typically carried out at reduced temperature in an inert solvent, for example in tetrahydrofuran, in the presence of a base, typically a pyridine derivative such as 2,6-lutidine. Formation of the phosphorane may be effected by treatment of the halo-intermediate with an appropriate phosphine derivative, for example tri-n-butylphosphine or triphenylphosphine, suitably at ambient temperature in an inert solvent such as dioxan.

A compound of formula (V) may be prepared by reaction of a compound of formula (VI): 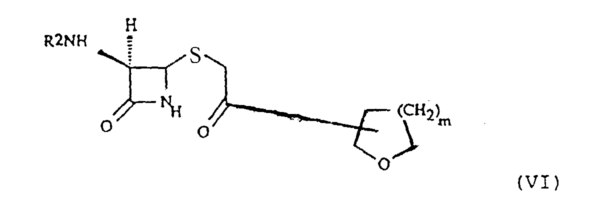 wherein R², and m are as hereinbefore defined with an ester of glyoxylic acid (OCHCO₂R¹) in the presence of triethylamine.

In a typical preparation of a compound of formula (VI) a compound of formula (VII): 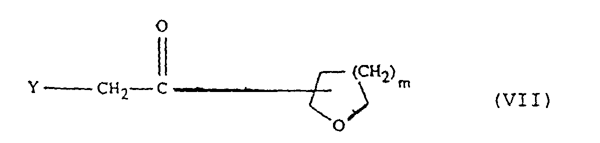 wherein Y is a leaving group and m is as hereinbefore defined is reacted with a compound of formula (VIII): 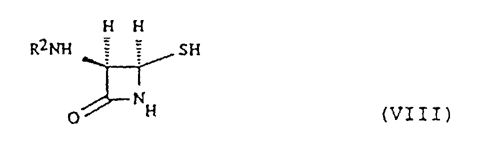 wherein R² is as hereinbefore defined.

Suitably, a leaving group Y is halogen, for example chloro. The reaction may be carried out at ambient temperature in an inert solvent, for example acetone or dimethylformamide, in the presence for a base, for example potassium carbonate.

A compound of formula (V) may also be prepared by reaction of a compound of formula (IX): 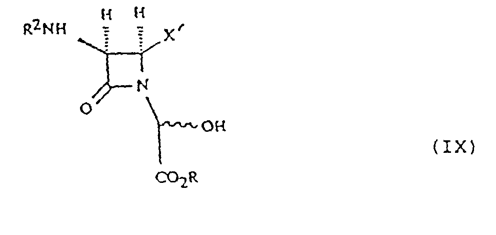 wherein R² and CO₂R¹ are as hereinbefore defined and X' is an S-group precursor, with a compound of formula (VII) as hereinbefore defined.

In a typical preparation of a compound of formula (V) a Y leaving group in a compound of formula (VII), suitably a halogen such as chloro or bromo, is displaced by an X' mercapto group in a compound of formula (IX). The reaction may be carried out at ambient temperature in an inert solvent, for example acetone, with the addition of base, for example potassium carbonate, before work-up.

Azetidin-2-one compounds of formulae (VIII) and (IX) may be prepared according to known methods in heterocyclic synthetic chemistry and particularly by known methods in the art of β-lactam chemistry. For example a compound of formula (VIII) may be prepared according to the method of Osborne N.F. et al., J. Chem. Soc., Perkin Trans. I, 146, 1980.

A compound of formula (IX) in which X' is a mercapto group may be prepared by ring opening of a 4-thia-2,6-diazabicyclo [3.2.0]-hept-2-ene-7-one derivative according to the method of Masayuki Narisada et al., Tetrahedron Lett., 1755 (1978).

Compounds of formula (VII) are known compounds or may be prepared by standard methodology. For example, the compounds of formula (VII) in which Y is chloro or bromo may be prepared from the corresponding carboxylic acid (Y=COOH) via formation of the acid chloride followed by treatment with diazomethane and reaction of the resulting diazo compound with hydrogen chloride or hydrogen bromide.

In a further process of the invention, compounds of formula (I) may be prepared directly by organo-cuprate displacement of a leaving group at the 3-position of a compound of formula (X): 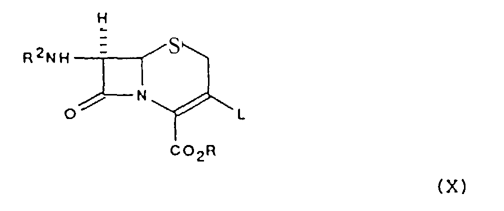 wherein R² and CO₂R¹ are as hereinbefore defined and L is a leaving group, suitably a mesylate, triflate or fluorosulphonate leaving group, by reaction with a compound of formula (XI): 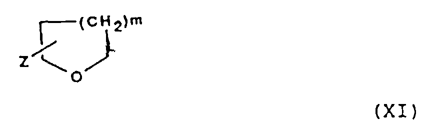 wherein Z is an organo-cuprate group and m are as hereinbefore defined.

A compound with a 3-position leaving group, L, may be prepared by the procedure of Farina V. et al., J. Org. Chem., 54, 4962, (1989).

It should be noted that in processes of this invention Δ ²-cephems may function as intermediates, in the synthetic sequences. Subsequent isomerisation steps by methods well known in cephalosporin chemistry will provide the Δ ³-cephems of the invention.

The present invention also provides a pharmaceutical composition which comprises a compound of formula (Ia) or a pharmaceutically acceptable salt or in vivo hydrolysable ester thereof and a pharmaceutically acceptable carrier. The compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral, especially oral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No unacceptable toxicological effects are expected when a compound of formula (Ia) or a pharmaceutically acceptable salt or in vivo hydrolysable ester thereof is administered in the above-mentioned dosage range.

The compound of formula (Ia) may be the sole therapeutic agent in the compositions of the invention or a combination with other antibiotics or with a β-lactamase inhibitor may be employed.

Advantageously, the compositions also comprise a compound of formula (XIII) or a pharmaceutically acceptable salt or ester thereof: 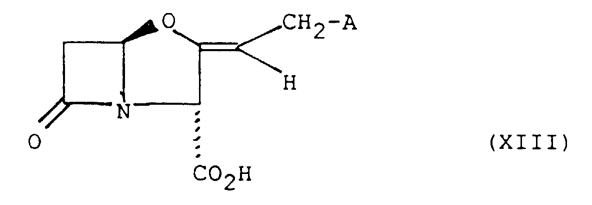 wherein
A is hydroxyl, substituted hydroxyl, thiol, substituted thiol, amino, mono- or di-hydrocarbyl- substituted amino, or mono- or di-acylamino; an optionally substituted triazolyl group; or an optionally substituted tetrazolyl group as described in EP-A-0 053 893.

A further advantageous composition comprises a compound of formula (Ia) or a pharmaceutically acceptable salt or in vivo hydrolysable ester thereof together with a compound of formula (XIV) or a pharmaceutically acceptable salt or in vivo hydrolysable ester thereof: 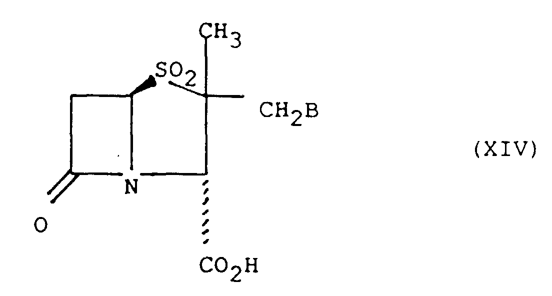 wherein
B represents hydrogen, halogen or a group of formula: 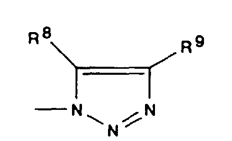 in which R⁸ and R⁹ are the same or different and each represents hydrogen, C₁₋₆ alkoxycarbonyl or carboxy, or a pharmaceutically acceptable salt thereof.

Further suitable β-lactamase inhibitors include 6-alkylidene penems of formula (XV): 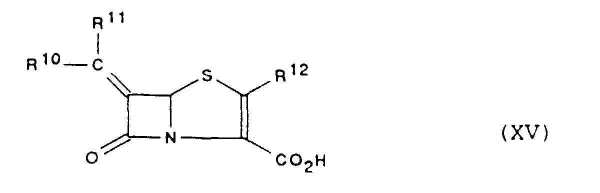 or a pharmaceutically acceptable salt or in vivo hydrolysable ester thereof, wherein R¹⁰ and R¹¹ are the same or different and each represents hydrogen, or a C₁₋₁₀-hydrocarbon or heterocyclic group optionally substituted with a functional group; and R¹² represents hydrogen or a group of formula R¹³ or -SR¹³ where R¹³ is an optionally substituted C₁₋₁₀ hydrocarbon or heterocyclic group, as described in EP-A-0 041 768.

Further suitable β-lactamase inhibitors include 6β-bromopenicillanic acid and pharmaceutically acceptable salts and in vivo hydrolysable esters thereof and 6β-iodopenicillanic acid and pharmaceutically acceptable salts and in vivo hydrolysable esters thereof described in, for example, EP-A-0 410 768 and EP-A-0 154 132 (both Beecham Group).

Such compositions of this invention which include a β-lactamase inhibitory amount of a β-lactamase inhibitor are formulated in a conventional manner using techniques and procedures per se known in the art.

The antibiotic compounds of the present invention are active against a wide range of organisms including both Gram-negative organisms such as E.coli and Gram-positive organisms such as S.aureus.

The following Examples illustrate the preparation of compounds of the invention and intermediates thereto. The following biological data illustrate the activity of compounds of the invention in the form of MIC values (minimum inhibitory concentration) against a sample E.coli organism (NCTC 10418) and a sample S.aureus organism (S.aureus Oxford).

### Example 1

### Sodium (6R,7R)-7-[2-(2-Aminothiazol-4-yl)-2-(Z)-methoxyiminoacetamido]-3-[(RS)-tetrahydrofuran-2-yl]ceph-3-em-4-carboxylate

### (a) (RS)-2-Chloroacetyltetrahydrofuran

Oxalyl chloride (5.2ml, 60mmol) and DMF (1 drop) were added to (RS)-2-tetrahydrofuroic acid (W.E. Kaufmann and R. Adams, J.Amer.Chem.Soc., 1923, 45, 3029) (4.64g, 40mmol) in dichloromethane (25ml). The mixture was stirred lh, evaporated in vacuo, dichloromethane added and reevaporated to give 2-tetrahydrofuroyl chloride, νₘₐₓ(CH₂Cl₂) 1795cm-¹. 2-Tetrahydrofuroyl chloride in ether (25ml) and dichloromethane (10ml) was added dropwise to an ice bath cooled solution of diazomethane (ca 80mmol) in ether (150ml). The reaction mixture was stirred 0.25h then a stream of hydrogen chloride gas passed into the solution for ca 2 minutes then stirred a further 0.25h, washed with saturated brine, dried, concentrated and flash chromatographed on silica gel eluting with 5,7.5 and 10% ethyl acetate in hexane to provide the title compound (2.46g, 41%); (Found: M⁺, 148.0279. C₆H₉ClO₂ requires M, 148.0291); νₘₐₓ(CH₂Cl₂) 1739, 1395, 1071 and 936cm⁻¹; δ_{H}(CDCl₃, 250MHz) 1.8-2.4 (4H, m) and 3.9-4.6 (5H, m).

### (b) (3R, 4R)-3-Phenoxyacetamido-4-[(RS)-tetrahydrofuran-2-ylcarbonylmethylthio]azetidin-2-one

(RS)-2-Chloroacetyltetrahydrofuran (2.46g, 16.5mmol), (3R,4R)-4-mercapto-3-phenoxyacetamidoazetidin-2-one (4.157g, 16.5mmol) and potassium carbonate (2.227g 16.5mmol) in DMF (10ml) were stirred for 2h, diluted with ethyl acetate, washed twice with water and with brine, dried concentrated and flash chromatographed eluting with 40,30,20,10 and 0% hexane in ethyl acetate to give the title compound as a foam (3.547g, 59%); νₘₐₓ(CH₂Cl₂) 3405, 1785, 1693, 1520, 1496 and 1240cm⁻¹; δ(CDCl₃, 250MHz) 1.9-2.3 (4H, m), 3.42 and 3.62, 3.46 and 3.56 (together 2H, 2 ABq, J15.8Hz, 15.4Hz), 3.85-4.0 (2H, m), 4.4-4.5 (1H, m), 4.58 (2H, s), 5.01, 5.04 (together 1H, 2d, J4.7Hz), 5.59 (1H, dd, J 8.8, 4.5Hz) 6.62, 6.68 (together 1H, 2s), 6.9-7.4 (5H, m) and 7.45, 7.47 (together 1H, 2d, J8.8Hz). [Mass spectrum: M⁺(364)].

### (c) t-Butyl (RS)-2-Hydroxy-2-[(3R, 4R)-3-phenoxyacetamido-4-[(RS)-tetrahydrofuran-2-ylcarbonylmethylthio]azetidin-2-on-1-yl]acetate

0.5M t-Butyl glyoxylate in 1,2-dichloroethane (20ml) and triethylamine (140µl, 1mmol) were added to (3R,4R)-phenoxyacetamido-4-[(RS)-tetrahydrofuran-2-ylcarbonylmethylthio]azetidin-2-one (3.547g, 9.7mmol) in 1,2-dichloroethane (10ml). The mixture was stirred 1h, concentrated in vacuo and flash chromatographed eluting with 50,60,70% ethyl acetate in hexane (3.663g, 76%); νₘₐₓ(CH₂Cl₂) 3471, 3407, 1782, 1736, 1692, 1521, 1290, 1154 and 1083cm⁻¹; δ_{H}(CDCl₃, 250MHz) 1.53 (9H, s), 1.85-2.25 (4H, m), 3.4-3.7 (2H, m), 3.8-4.0 (2H, m), 4.3-4.45 (1H, m), 4.57 (2H, s), 5.07, 5.09, 5.16, 5.18 (together 1H, 4d, J4.8Hz), 5.25-5.45 (1H, m), 5.48, 5.58 (together 1H, 2dd, J4.8, 8.8Hz), 6.9-7.4 (5H, m) and 7.41, 7.56 (together 1H, 2d, J8.7Hz). [Mass spectrum: +ve ion (thioglycerol) MH⁺(495)].

### (d) t-Butyl 2-[(3R,4R)-3-Phenoxyacetamido-4-[(RS)-tetrahydrofuran-2-ylcarbonylmethylthio]azetidin-2-on-1-yl]-2-tri-n-butylphosphoranylideneacetate

Thionyl chloride (0.81ml, 11.1mmol) in THF (5ml) was added dropwise to the hydroxy compound (3.663g, 7.4mmol) and 2,6-lutidine (1.29ml, 11.1mmol) in THF (15ml) at -20°C. The mixture was stirred 0.5h, filtered and the filtrate evaporated in vacuo, toluene added and re-evaporated to give t-butyl (RS) -2-chloro-2-[(3R,4R)-3-phenoxyacetamido-4-[(RS)-tetrahydrofuran-2-ylcarbonylmethylthio]azetidin-2-on-1-yl]acetate as a foam (4.222g).

To the crude chloro compound in dioxan (10ml) was added tri-n-butylphosphine (4.06ml, 16.3mmol), the solution stirred 0.75h, [Bdiluted with ethyl acetate, washed with dilute sodium hydrogen carbonate solution, water and brine, dried concentrated and flash chromatographed on silica gel eluting with 30,40,50,60,70,80% ethyl acetate in hexane to give the title compound as a foam (3.827g, 76%); νₘₐₓ(CH₂Cl₂) 3417, 1764, 1731, 1690, 1628, 1523, 1171 and 1082cm⁻¹ [Mass spectrum: +ve ion (thioglycerol) MH⁺ (679)].

### (e) t-Butyl (6R,7R)-7-Phenoxyacetamido-3-[(RS)-tetrahydrofuran-2-yl] ceph-3-em-4-carboxylate

The phosphorane (3.827g) and benzoic acid (20mg) in toluene (75ml) were purged with argon then heated under argon in an oil bath at 130°C for 6h. The solution was left to cool and flash chromatographed on silica gel eluting with 30% ethyl acetate in hexane to give the title compound as a foam (2.267g, 87%); νₘₐₓ(CH₂Cl₂) 3406, 1785, 1697, 1519, 1155 and 1054cm⁻¹; δ_{H}(CDCl₃, 250MHz) 1.53, 1.54 (together 9H, 2s), 1.5-2.5 (4H, m), 3.29 and 3.61, 3.39 and 3.56 (together 2H, 2ABq, J18.6, 18.0Hz), 3.8-4.0 (2H, m), 4.57 (2H, s), 4.9-5.0, 5.05-5.2 (together 1H, 2m), 5.01, 5.02 (together 1H, 2d, J4.8Hz), 5.84, 5.91 (together 1H, 2dd, J4.8, 9.4Hz) and 6.9-7.4 (6H, m). [Mass spectrum +ve ion (3-nitrobenzyl alcohol, sodium acetate) MNa⁺ (483)].

### (f) t-Butyl (6R,7R)-7-Amino-3-(tetrahydrofuran-2-yl)ceph-3-em-4-carboxylate

Phosphorus pentachloride (1.538g, 7.5mmol) in dichloromethane (39ml) was added to t-butyl(6R, 7R)-7-phenoxyacetamido-3-[(RS)-tetrahydrofuran-2-yl]ceph-3-em-4-carboxylate (2.267g, 4.9mmol) and N-methylmorpholine (1.1ml, 10mmol) in dichloromethane (20ml) at -25°C. The reaction was stirred at -10±5°C for 0.75h then methanol (10ml) added all at once, stirred 0.75h then water (20ml) added and stirred vigorously for lh. The dichloromethane was evaporated in vacuo, the aqueous residue washed with ether then adjusted to pH7 with ammonium hydroxide in the presence of ethyl acetate. The mixture was extracted twice with ethyl acetate, the extracts dried, concentrated and flash chromatographed on silica gel eluting with 30,40,50% ethyl acetate in hexane to give the more mobile (S)-diastereoisomer of the title compound (0.431g, 27%); (Found: M⁺, 326.1299. C₁₅H₂₂N₂O₄S requires M, 326.1300); νₘₐₓ(CH₂Cl₂) 1777, 1716, 1158 and 1052cm⁻¹; δ(CDCl₃, 250MHz) 1.52 (9H,s), 1.55-1.8 (1H, m), 1.85-2.05 (4H, m), 2.3-2.45 (1H, m), 3.30 and 3.59 (2H, ABq, J18.4Hz), 3.8-4.0(2H,m), 4.75 (1H, d, J5.0Hz) and 4.9-5.0 (2H, m). Further elution with 60% ethyl acetate in hexane gave the more polar (R)-diastereoisomer (0.533g, 33%); (Found: M⁺ 326.1299. C₁₅H₂₂N₂O₄S requires M, 326.1300) νₘₐₓ (CH₂Cl₂) 1776, 1721, 1158 and 1052cm⁻¹; δ_{H}(CDCl₃, 250MHz) 1.41 (2H, bs), 1.54 (9H, s), 1.6-1.85 (1H, m), 1.9-2.05 (2H, m), 2.05-2.2 (1H, m), 3.40 and 3.55 (2H, ABq, J17.8Hz) 3.8-4.0 (2H, m), 4.67 (1H, d, J5.0Hz), 4.93 (1H, d, J5.0Hz), 5.0-5.15 (1H, m).

### (g) t-Butyl (6R,7R)-7-[2-(z)-Methoxyimino-2-(2-tritylaminothiazol-4-yl)acetamido]-3-(tetrahydrofuran-2-yl)ceph-3-em-4-carboxylate

Mesyl chloride (141µl, 1.8mmol) was added to 2-(Z)-methoxyimino-2-(2-tritylaminothiazol-4-yl)acetic acid hydrochloride (0.744g, 1.65mmol) and N,N-diisopropylethylamine (576µl, 3.3mmol) in DMF (5ml) at -40°C. The reaction mixture was stirred 0.5h at -30±10°C then t-butyl (6R, 7R) -7-amino-3-(tetrahydrofuran-2-yl)ceph-3-em-4-carboxylate, more mobile diastereoisomer (0.431g, 1.3mmol) in DMF (5ml) followed by pyridine (147µl, 1.8mmol) were added. Stirred lh without further cooling then diluted with ethyl acetate, washed twice with water and with brine, dried, concentrated and flash chromatographed on silica gel eluting with 30,35 and 40% ethyl acetate in hexane to give the title compound as a foam (0.83g, 84%); νₘₐₓ(CH₂Cl₂) 3396, 3277, 1782, 1732, 1683, 1526, 1248, 1156 and 1051cm⁻¹; δ_{H}[(CD₃)₂SO, 250MHz] 1.47 (9H, s), 1.55-1.75 (1H, m), 1.8-2.0 (2H, m), 2.05-2.2 (1H, m), 3.44 and 3.50 (2H, ABq, J18.3Hz), 3.65-3.95 (2H, m), 3.81 (3H, s), 4.6-4.7 (1H, m), 5.14 (1H, d, J4.8Hz), 5.66 (1H, dd, J4.8, 7.9Hz), 6.70 (1H, s), 7.2-7.4 (15H, m), 8.88 (1H, s) and 9.54 (1H, d, J7.9Hz). [Mass spectrum: +ve ion (3-nitrobenzyl alcohol, sodium acetate) MNa⁺ + (774)].

### (h) Sodium (6R-7R)-7-[2-(2-Aminothiazol-4-yl)-2-(Z)-methoxyiminoacetamido]-3-[(RS)-tetrahydrofuran-2-yl]ceph-3-em-4-carboxylate

t-Butyl (6R,7R)-7-[2-(Z)-methoxyimino-2-(2-tritylaminothiazol-4-yl)acetamido]-3-(tetrahydrofuran-2-yl)ceph-3-em-4-carboxylate, single diastereoisomer (0.832g, 1.1mol) in 0.1M hydrochloric acid in 90% formic acid (11ml) was stood for lh, concentrated hydrochloric acid (200µl) added and left for a further 1.5h then evaporated to dryness in vacuo. The residue in water (ca 5ml) was adjusted to pH6.5 with 1M sodium hydroxide solution and chromatographed on HP20SS eluting with 0,1,2 and 3% THF in water. Fractions containing the product, h.p.l.c. analysis, were combined, concentrated and freeze dried to give the title compound as a mixture of diastereoisomers (271mg, 52%); νₘₐₓ(KBr) 1762, 1669, 1603, 1530, 1388 and 1039cm⁻¹; δ_{H}[(CD₃)₂SO, 250MHz] 1.4-2.05 (4H, m), 3.19 and 3.36, 3.26 and 3.83 (together 2H, 2ABq, J17.5, 16.8Hz), 3.55-3.85 (1H, m), 3.83 (3H, s), 4.85-4.95, 5.15-5.25 (together 2H, 2m), 4.96, 4.97 (together 1H, 2d, J4.7Hz), 5.49, 5.53 (together 1H, 2dd, J4.7, 7.9Hz), 6.74, 6.75 (together 1H, 2s), 7.24 (2H, s), 9.49, 9.52 (together 1H, 2d, J7.9Hz) [Mass spectrum +ve ion (thioglycerol) MH⁺ (476), MNa⁺ (498)].

The same mixture of diastereoisomers was obtained by progressing the other diastereoisomer isolated in stage (f).

### Example 2

### Pivaloyloxymethyl (6R,7R)-7-[2-(2-Aminothiazol-4-yl)-2-(Z)-methoxyiminoacetamidol-3-[(RS)-tetrahydrofuran-2-yl]ceph-3-em-4-carboxylate

Pivaloyloxymethyl bromide (0.15g) and sodium iodide (0.15g) in acetone (lml) were stirred 0.5h, filtered and the filtrate evaporated to give the iodide. This in toluene (0.5ml) was added to sodium (6R,7R)-7-[2-(-2-aminothiazol-4-yl)-2-(Z)-methoxyiminoacetamido]-3-[(RS)-tetrahydrofuran-2-yl]ceph-3-em-4-carboxylate (0.191g) in N-methylpyrrolidone (1ml) and stirred 0.5h. The reaction mixture was diluted with ethyl acetate, washed twice with water and with brine, dried, concentrated and flash chromatographed on silica gel eluting with 80% ethyl acetate in hexane to give the title compound (130mg, 57%); νₘₐₓ(CH₂Cl₂) 3478, 3391, 1787, 1752, 1685, 1125, 1098 and 1052cm-1; δ_{H}(CDCl₃, 250MHz), 1.23 (9H,s), 1.6-2.5 (4H, m), 3.37 and 3.66, 3.43 and 3.62 (together 2H, 2ABq, J18.8, 17.8Hz), 3.8-4.05 (2H, m), 4.10 (3H, s), 4.85-5.0, 5.15-5.25 (together 1H, 2m), 5.07, 5.08 (together 1H, 2d, J4.8, 4.7Hz), 5.8-6.05 (3H, m), 6.95, 6.96 (together 1H, 2s) and 7.54, 7.65 (together 1H, 2d, J8.8, 8.5Hz). [Mass spectrum: +ve ion (thioglycerol) MH⁺ 568)].

### Example 3

### Sodium (6R,7R)-7-[2-(2-Aminothiazol-4-yl)-2-(Z)-methoxyiminoacetamido]-3-[(RS)-tetrahydropyran-2-yl]ceph-3-em-4-carboxylate

### (a) Tetrahydropyran-2-carboxylic acid

3,4-Dihydro-2H-pyran-2-carboxylic acid, sodium salt (5.0g) in water (30ml) was treated with 10% Palladium on carbon catalyst (0.2g) and the mixture hydrogenated until there was no further uptake of hydrogen. The mixture was filtered through Kieselguhr, the filtrate passed through a column of 'Amberlite IR120(H⁺), evaporated in vacuo and the residue dissolved in dichloromethane, dried and evaporated to give the title compound as colourless oil. (3.3g, 76%); (Found: M⁺, 130.0631. C₆H₁₀O₃ requires M, 130.0630; ν_{maX}(CH₂Cl) 3500-2750 (v.br), 1772, 1725cm⁻¹; δ_{H}(CDCl₃), 1.5-1.7 (4H, m), 1.8-2.1 (2H, m), 3.50-3.59 (1H, m), 3.99-4.14 (2H, m) and 7.28 (1H, br.s).

### (b) 2-(2-Chloroacetyl)tetrahydropyran

Tetrahydropyran-2-carboxylic acid (3.3g) in dry dichloromethane (60ml) was treated with oxalyl chloride (4.8g, 3.3ml) and DMF (2-3 drops). After the initial effervescence had ceased the mixture was left for a further lh at ambient temperature. The solvent and excess oxalyl chloride were removed in vacuo and the resultant oil [νₘₐₓ(CH₂Cl₂)1830cm⁻¹] was dissolved in dichloromethane (20ml). This acid chloride solution was then added dropwise to a freshly prepared ethereal solution of diazomethane (ca 2 fold excess) cooled to 0-5°C, t.l.c. analysis (60% ethyl acetate in hexane) showed a single mobile spot, i.r. spectrum of a sample showed clean conversion to the diazoketone [νₘₐₓ (CH₂Cl₂) 2100cm⁻¹]. Hydrogen chloride gas was bubbled through the solution until no further starting material was observed by t.l.c. The mixture was washed with brine, dried and the solvent removed in vacuo and the residue purified by flash chromatography on silica gel. The title compound was obtained as a pale yellow oil, (2.8g, 68%); νₘₐₓ(CH₂Cl₂) 1740cm⁻¹; δ_{H}(CDCl₃) 1.4-1.7 (4H, m), 1.91-1.98 (2H, m), 3.42-3.53 (1H, m), 3.95-4.07 (2H, m) and 4.48 (2H, s) [Mass spectrum: +ve ion (NH₃), MH⁺ (163), MNH₄⁺ (180)].

### (c) (3R, 4R)-3-Phenylacetamido-4-[(RS)-tetrahydropyran-2-ylcarbonylmethylthio]azetidin-2-one

3R,4R-Mercapto-3-phenylacetamidoazetidin-2-one (2.6g) and 2-(2-chloroacetyl)tetrahydropyran (1.6g) in DMF (20ml) were treated with potassium carbonate (1.6g) at ambient temperature for ca 2h until t.l.c. (80% ethylacetate in hexane) showed loss of starting material. The reaction mixture was diluted with ethyl acetate,washed with water (x3), brine, dried and concentrated. The title compound was obtained by flash chromatography (60%,70% ethyl acetate in hexane, ethyl acetate) as a mixture of diastereoisomers as a colourless foam (1.7g, 70%); νₘₐₓ(CH₂Cl₂), 3380(w), 1783, 1726, 1684cm⁻¹; δ_{H}(CDCl₃) 1.3-1.7 (4H, m), 1.8-2.0 (2H, m), 3.3-3.6 (3H, m), 3.66 (2H, s), 3.86-3.90 (1H, m), 4.03-4.07 (1H, m), 4.92 (1H, d, J4.6Hz), 5.51 (1H, dd, J4.4, 8.6Hz) 6.42 (d, J8.7Hz), 6.48, 6.51 (together 1H, 2s) and 7.27-7.36 (5H, m). [Mass spectrum: M⁺ (362)].

### (d) t-Butyl (RS)-2-Hydroxy-2-[(3R, 4R)-3-phenylacetamido-4-[(RS)-tetrahydropyran-2-ylcarbonylmethylthio]azetidin-2-on-1-yl]acetate

(3R,4R)-3-Phenylacetamido-4-[(RS)-tetrahydropyran-2-yl-carbonylmethylthio]azetidin-2-one (1.7g) in 1,2-dichloroethane (20ml) was successively treated with 0.5M t-butyl glyoxylate in 1,2-dichlorethane (10ml) and triethylamine (50mg, 70µl) and monitored by t.l.c. (ethyl acetate) until no starting material remained. The reaction mixture was concentrated and flash chromatography (70% ethyl acetate in hexane, ethyl acetate) to afford the title compound as a yellow foam (1.9g, 82%); νₘₐₓ (CH₂Cl₃) 3400 (w) , 1780, 1736, 1687cm⁻¹; δ_{H}(CDCl₃) 1.49 (9H, s) overlapping 1.44-1.61 (4H, m), 1.8-2.0 (2H, m), 3.35-3.58 (3H, m), 3.65 (2H, s), 3.81-3.92 (1H, m), 4.01-4.06 (1H, m), 4.28-4.43 (1H, m), 4.99, 5.00, 5.07 (together 1H, 3d, J4.7Hz), 5.21, 5.32, 5.33 (together 1H, 3d, J6.8, 7.7, 7.6Hz), 5.42, 5.50 (together 1H, 2dd, J4.8, 8.7Hz, 6.35, 6.36, 6.61 (together 1H, 3d, J8.7Hz) and 7.27-7.38 (5H, m).

### (e) t-Butyl 2-[(3R,4R)-3-Phenvlacetamido-4-[(RS)-tetrahydropyran-2-ylcarbonylmethylthio]azetidin-2-on-1-yl]-2-tri-n-butylphosphoranylideneacetate

t-Butyl 2-hydroxy-2-[(3R, 4R) -3-phenylacetamido-4-[(RS)-tetrahydropyran-2-ylcarbonylmethylthio]azetidin-2-on-1-yl]acetate (1.9g) in dry THF (10ml) was treated with 2,6-lutidine (0.62g, 0.67ml) followed by thionyl chloride (0.69g, 0.42ml) in THF (5ml) dropwise at <-20°C under argon. The reaction mixture was allowed to warm slowly to ca 0°C at which point no starting material was observed by t.l.c. (ethyl acetate) . The reaction mixture was filtered and solvent removed in vacuo, the residue dissolved in toluene and evaporated to afford crude t-butyl (RS)-2-chloro-2- [(3R, 4R) -3-phenylacetamido-4-[(RS) -tetrahydropyran-2-ylcarbonylmethylthio]azetidin-2-on-1-yl]acetate as a brown gum. This was dissolved in dry dioxan (10ml) and treated with tri-n-butylphosphine (1.79g, 2.2ml). The reaction mixture was stirred until loss of starting material was observed by t.l.c. (ethyl acetate) ca 0.5h. After removal of solvent in vacuo the title compound was obtained by flash chromatography (eluting with 50,60,80% ethyl acetate in hexane, ethyl acetate) as a pale brown foam (1.95g, 75%); νₘₐₓ(CH₂Cl₂) 3417(w), 1762, 1681, 1625cm⁻¹. [Mass spectrum +ve ion (thioglycerol) MH⁺ (677)].

### (f) t-Butyl (6R, 7R)-7-Phenylacetamido-3-[(RS)-tetrahydropyran-2-yl]ceph-3-em-4-carboxylate

t-Butyl 2-[(3R, 4R)-3-phenylacetamido-4-[(RS)-tetrahydropyran-2-yl]carbonylmethylthio]azetidin-2-on-1-yl]-2-tri-n-butylphosphoranylideneacetate (1.95g) in dry toluene (50ml) was refluxed for 8h under argon. The solvent was removed in vacuo and the title compound obtained by flash chromatography (30% ethyl acetate in dichloromethane) as a yellow foam (1.15g, 87%); νₘₐₓ(CH₂Cl₂), 3415(w), 1783, 1721, 1687cm⁻¹; δ_{H}(CDCl₃), 1.54, 1.56 (together 9H, 2s) overlapping 1.46-1.68 (4H, m), 1.76-1.94 (2H, m), 3.42-3.68 (5H, m), 3.97-4.06 (2H, m), 4.52-4.65 (1H, m), 4.98 (1H, d, J4.8Hz), 5.68, 5.71 (together 1H, dd, J4.7) and 7.23-7.36 (5H, m). [Mass spectrum: +ve ion (3-nitrobenzyl alcohol, sodium acetate) MNa⁺ (481)].

### (g) t-Butyl (6R,7R)-7-Amino-3-[(RS)-tetrahydropyran-2-yl]ceph-3-em-4-carboxylate

t-Butyl (6R, 7R) -7-phenylacetamido-3-[(RS)-tetrahydropyran-2-yl]ceph-3-em-4-carboxylate (1.1g) in dry dichloromethane (50ml) at -20°C under argon was successively treated with N-methylmorpholine (0.55g, 0.6ml) and phosphorus pentachloride (0.65g as 16.25ml of a 40mg/ml solution in dry dichloromethane) and stirred at -20°C for 0.75h. Methanol (50ml) was added and reaction mixture allowed to warm to ambient temperature over a period of ca 0.5h. Water (50ml) was added and reaction mixture stirred vigourously for a further 0.5h. The dichloromethane was removed in vacuo, ethyl acetate added and aqueous layer adjusted to pH8 with 0.880 ammonia and reextracted with ethyl acetate. The organic extracts were washed with water, brine, dried, concentrated and flash chromatographed on silica gel (eluting with 70,80% ethyl acetate in hexane, ethyl acetate). The first isomer to be eluted (isomer A) was obtained as a white foam (300mg, 37%); νₘₐₓ (CH₂Cl₂) 1776, 1717cm⁻¹; δ_{H}(CDCl₃), 1.53 (9H, s) overlapping 1.4-1.7 (4H, m), 1.73-1.97 (2H, m), 3.35-3.55 (lH, m) overlapping 3.49 and 3.55 (2H, ABq, J18.4Hz), 3.96-4.00 (1H, m), 4.51-4.55 (1H, m), 4.72 (1H, d, J5.0Hz) and 4.93 (1H, d, J5.0Hz). [Mass spectrum: M⁺ (340)]. The second isomer to be eluted (isomer B) was obtained as a white foam (400mg, 49%); νₘₐₓ(CH₂Cl₂), 1715, 1721cm⁻¹; δ_{H}(CDCl₃) 1.56 (9H, s) overlapping 1.49-1.66 (4H, m), 1.84-2.05 (2H, m), 3.44 and 3.62 (2H, ABq, J 17.8) overlapping 3.45-3.54 (1H, m), 4.01-4.11 (1H, m), 4.56-4.61 (1H, m), 4.69 (1H, d, J5.0Hz) and 4.93 (1H, d, J5.0Hz). [Mass spectrum: M⁺ (340)].

### (h) t-Butyl (6R, 7R)-7-[2-(Z)-Methoxyimino-2-(2-tritylaminothiazol-4-yl)acetamido]-3-[tetrahydropyran-2-yl]ceph-3-em-4-carboxylate

Mesyl chloride (121mg, 82µl) was added to 2-(Z)-methoxyimino-2-(2-tritylaminothiazol-4-yl)acetic acid hydrochloride (466mg) and N,N-diisopropylethylamine (252mg, 340µl) in dry DMF (10ml) at -50°C under argon and stirred at -50°C for lh. Then t-butyl (6R, 7R) -7amino-3-(tetrahydropyran-2-yl)-ceph-3-em-4-carboxylate (Isomer A, 300mg) in dry DMF (5ml) followed by pyridine (70mg, 72µl) were added and reaction mixture left for a further lh whilst warming to ambient temperature. The reaction mixture was partitioned between ethyl acetate and water, reextracted with ethyl acetate, organic extracts washed with water (x3) and brine, dried, concentrated and flash chromatography (eluting with 30,40,50,60% ethyl acetate in hexane) to afford the title compound as a pale yellow foam (420mg, 62%); νₘₐₓ(CH₂Cl₂) 3420, 1784, 1732 (shoulder), 1717, 1685cm⁻¹; δ_{H}(CDCl₃), 1.53 (9H, s) overlapping 1.4-1.7 (4H, m), 1.73-1.94 (2H, m), 3.38-3.58 (3H, m), 3.95-4.00 (lH, m), 4.07 (3H, s), 4.54-4.59 (1H, m), 5.02 (1H, d, J4.8Hz), 5.90 (1H, dd, J4.5, 9.1Hz) 6.74 (1H, s), 6.86 (1H, d, J8.8Hz), 7.04 (1H, s) and 7.30 (15H, s). [Mass spectrum: +ve ion (3-nitrobenzyl alcohol, sodium acetate) MNa⁺ (788)].

### (i) Sodium (6R,7R)-7-[2-(2-Aminothiazol-4-yl)-2-(Z)-methoxyiminoacetamido]-3-[(RS)-tetrahydropyran-2-yl]ceph-3-em-4-carboxylate

t-Butyl (6R,7R)-7-[2-(Z)-methoxyimino-2-(2-tritylaminothiazol-4-yl)acetamido]-3-(tetrahydropyran-2-yl)ceph-3-em-4-carboxylate (400mg) was dissolved in 0.1M hydrochloric acid in 90% formic acid (5.22ml) and set aside for 0.5h, concentrated hydrochloric acid (50µl) added and left for a further 1.5h. The mixture was evaporated in vacuo, diluted with water, adjusted to pH6.7 with sodium bicarbonate, then chromatographed on HP20SS eluting with water then 1,2,4,6,8% THF in water. Fractions containing a diastereoisomeric mixture of the title compound (h.p.l.c.) were concentrated in vacuo and freeze dried (170mg, 66%); νₘₐₓ(KBr) 1770, 1670, 1600, 1535cm⁻¹; δ_{H}[(CD₃)₂SO], 1.3-1.5 (4H, m), 1.6-1.85 (2H, m), 3.24-3.44 (m, masked by HOD peak), 3.83 (3H, s) overlapping 3.76-3.95 (1H, m), 4.46-4.50, 4.82-4.86 (together 1H, 2m), 4.94 (1H, d, J4.7Hz), 5.46-5.53 (1H, m), 6.74, 6.75 (together 1H, 2s), 7.23 (2H, s) and 9.48, 9.51 (together 1H, 2d, J5.6, 5.5Hz). [Mass spectrum: +ve ion (thioglycerol) MH⁺ (490), MNa⁺ (512)].

The second isomer eluted (isomer B) in step (g) (400mg) was progressed through step (h) as before yielding a pale yellow foam (550mg, 61%); νₘₐₓ(CH₂Cl₂), 3420, 1783, 1729, 1687cm⁻¹; δ_{H}(CDCl₃) 1.55 (9H, s) overlapping 1.44-1.68 (4H, m), 1.82-1.96 (2H, m), 3.44 and 3.65 (2H, ABq, J18.0) overlapping 3.42-3.58 (1H, m), 4.07 (3H, s) overlapping 3.96-4.10 (1H, m), 4.66-4.69 (1H, m), 4.66-4.69 (1H, m), 5.01 (1H, d, J4.7Hz), 5.86 (1H, dd, J4.8, 8.9Hz), 6.75 (1H, s) overlapping 6.75-6.78 (1H, m), 7.01 (1H, s) and 7.30 (15H, s). [Mass spectrum: +ve ion (3-nitrobenzyl alcohol, sodium acetate) MNa⁺ (788)]. This was then progressed through step (i) to afford the same mixture of diastereoisomers.

### Example 4

### Pivaloyloxymethyl (6R,7R)-7-[2-(2-Aminothiazol-4-yl)-2-(Z)-methoxyiminoacetamido]-3-[(RS)-tetrahydropyran-2-yl]ceph-3-em-4-carboxylate

The title compound was prepared from the compound of Example 3 as described in Example 2 and obtained as a pale yellow foam (59%); ν_{maX}(CH₂Cl₂); 3388, 1787, 1752, 1688cm⁻¹; δ_{H}(CDCl₃) 1.24 (9H, s), 1.42-1.64 (4H, m), 1.74-1.90 (2H, m), 3.40-3.75 (3H, m), 4.07, 4.08 (together 3H, 2s) overlapping 3.96-4.10 (1H, m), 4.56-4.59, 4.80-4.83 (together 1H, 2m), 5.07, 5.08 (together 1H, 2d, J4.8, 4.7Hz), 5.66 (2H, br.s), 5.85-6.03 (3H, m), 6.86, 6.89 (together 1H, 2s) and 7.59 (1H, d, J8.9Hz). [Mass spectrum: +ve ion (thioglycerol) MH⁺ (582); MNa⁺ (604)].

### Example 6 (preparative)

### Diastereoisomers of (6R,7R) -7-Amino-3-(tetrahydrofuran-2-yl) ceph-3-em-4-carboxylate

### (a) (RS)-2-Bromoacetyltetrahydrofuran

A stream of diazomethane (from N-methyl-N-nitrosotoluene-4-sulphonamide, 18.0g) in argon (P. Lombardi, Chem. and Ind., 1990, (21), 708) was passed into a solution of (RS)-tetrahydrofuroyl chloride [prepared from (RS)-tetrahydrofuroic acid (3.48g, 30mmol) as described in Example 2(a)] in dichloromethane (60ml) cooled in an ice bath. When the diazomethane addition was complete, 48% aqueous hydrogen bromide (5.6ml, 33.2mmol) was added. The mixture was stirred 0.25h then washed twice with water, dried, concentrated and flash chromaographed on silica gel eluting with 10% ethyl acetate in hexane to give the title compound as a pale yellow oil (4.44g, 77%); νₘₐₓ (CH₂Cl₂) 1733, 1245, 1073 and 938cm⁻¹; δ_{H} (CDCl₃) 1.85-2.35 (4H, m), 3.85-4.05 (2H, m), 4.20 (2H, s), 4.54 (1H, dd, J 6.1, 8.2Hz). [Mass spectrum: +ve ion (ammonia) MNH₄⁺ (210)].

### (b) 4-Methoxybenzyl (2RS)-2-hydroxy-2-[(3R,4R)-3-phenylacetamido-4-[(RS)-tetrahydrofuran-2-yl-carbonylmethylthio]azetidin-2-on-1-yl]acetate

Toluene-4-sulphonic acid (6.0g, 31.5mmol) in water (15ml) was added to a solution of 4-methoxybenzyl (2RS)-2-hydroxy-2-[(1R, 5R)-3-benzyl-4-thia-2,6-diazabicyclo [3.2.0]hept-2-en-7-on-6-yl]acetate (7.42g, 18.0mmol) prepared from Penicillin G as described for the benzhydryl ester derived from Penicillin V, S. Yamamoto, N. Haga, T. Aoki, S. Hayashi, H. Tanida, and W. Nagata, Heterocycles, 1977, 8, 283) in dichloromethane (30ml) and acetone (30ml). After stirring for 2.5h at room temperature, the reaction mixture was diluted with dichloromethane, washed with water (x2), dried and concentrated in vacuo to yield crude 4-methoxybenzyl (2RS)-2-hydroxy-2-[(3R,4R) -4-mercapto-3-phenylacetamidoazetidin-2-on-1-yl]acetate as a yellow foam.

The crude thiol was dissolved in acetone (35ml) and treated with a solution of (RS)-2-bromoacetyltetrahydrofuran (3.48g, 18.0mmol) in acetone (5ml). After 10min, potassium carbonate (1.24g, 8.9mmol) was added, and the mixture stirred for a further 30min. The reaction mixture was diluted with ethyl acetate, washed successively with water (x2) and brine, dried and concentrated. The residue was flash chromatographed on silica gel eluting with 50, 70 and 80% ethyl acetate in hexane yielding the title compound as a colourless foam (5.40g, 55%); νₘₐₓ (CH₂Cl₂) 3409, 1781, 1745, 1684, 1613, 1516cm⁻¹. [Mass spectrum +ve ion (3-nitrobenzyl alcohol, sodium acetate) MNa⁺ (565)].

### (c) 4-Methoxybenzyl 2-[(3R,4R)-3-phenylacetamido-4-[(RS)-tetrahydrofuran-2-ylcarbonylmethylthio]azetidin-2-on-1-yl]-2-tri-n-butylphosphoranylideneacetate

A solution of thionyl chloride (1.36ml, 18.6mmol) in THF (10ml) was added dropwise to the hydroxy compound (6.72g, 12.4mmol) and 2,6-lutidine (2.16ml, 18.6mmol) in THF (30ml) at -20°C. After stirring for lh, the reaction mixture was filtered through a pad of celite, and the filtrate evaporated in vacuo. Toluene was added and re-evaporated to yield 4-methoxybenzyl (RS)-2-chloro-2-[(3R,4R)-3-phenylacetamido-4-[(RS)-tetrahydrofuran-2-ylcarbonylmethylthio]azetidin-2-on-1-yl]acetate as an oil.

The crude chloro compound was dissolved in dioxan (30ml) and treated with tri-n-butylphosphine (6.8ml, 27.3mmol). After stirring for 30min. at room temperature, the reaction mixture was diluted with ethyl acetate and washed successively with dilute sodium hydrogen carbonate solution, water and brine. The organic solution was dried, concentrated and then flash chromatographed on silica gel eluting with 50, then 80% ethyl acetate in hexane to give the title compound as a foam (6.54g, 73%); νₘₐₓ (CH₂Cl₂) 3422, 1763, 1732, 1680, 1613, 1515, 1174cm⁻¹. [Mass spectrum +ve ion/thioglycerol) MH⁺ (727), MNa⁺ (749)].

### (d) 4-Methoxybenzyl (6R,7R)-7-phenylacetamido-3-[(RS)-tetrahydrofuran-2-yl] ceph-3-em-4-carboxylate

A solution of the phosphorane (6.40g, 8.82mmo1) and benzoic acid (20mg) in toluene (100ml) was heated in an oil bath at 130°C for 10h under argon. The reaction mixture was cooled, concentrated and the residue purified by chromatography on silica gel eluting with 20, 30, 40, 50% ethyl acetate in hexane yielding the title compound as a yellow oil (3.50g, 78% yield); νₘₐₓ (CH₂Cl₂) 3411, 1783, 1723, 1688, 1515cm⁻¹; δ_{H} (CDCl₃, 250MHz) 1.50-2.39 (together 4H, m), 3.27 and 3.60, 3.32 and 3.49 (together 2H, 2ABq, J 18.7, 17.9Hz), 3.58 and 3.70, 3.63 and 3.73 (together 2H, 2ABq, J 16.2, 16.1Hz), 3.80, 3.82 (together 3H, 2S), 3.84-3.97 (together 2H, 2m), 4.91, 5.18 (together 1H, 2m), 4.90, 4.94 (together 1H, 2d, J 4.7Hz), 5.17 (2H, s), 5.73, 5.82 (together 1H, 2dd, J 9.1, 4.7Hz), 5.98, 6.02 (together lH, 2d, J 9.1Hz), 6.87 and 6.90 (together 2H, 2d, J 8.6Hz), 7.23-7.40 (7H, m). [Mass spectrum +ve ion (3-nitrobenzylalcohol, sodium acetate) MNa⁺ (531)].

### (e) 4-Methoxybenzyl (6R,7R)-7-amino-3-(tetrahydrofuran-2-yl)ceph-3-em-4-carboxylate

Phosphorus pentachloride (2.15g, 10.32mmol) in dichloromethane (108ml) was added to 4-methoxybenzyl (6R, 7R) -7-phenylacetamido-3-[(RS)-tetrahydrofuran-2-yl]ceph-3-em-4-carboxylate (3.40g, 6.69mmol) and N-methylmorpholine (1.50ml, 13.64mmol) in dichloromethane (30ml) at -25°C. The reaction was stirred at -10±5°C for 45min., then methanol (14ml) was added, and stirring continued for 45min. at room temperature. Water (27ml) was then added, and the mixture vigorously stirred for a further lh. The dichloromethane was evaporated in vacuo, the aqueous residue washed with diethyl ether, then adjusted to pH7 with ammonium hydroxide in the presence of ethyl acetate. The mixture was extracted with ethyl acetate (x2), dried and concentrated in vacuo. The residue was purified by chromatography on silica gel eluting with 50, 70, 80% ethyl acetate in hexane yielding 4-methoxybenzyl (6R,7R) -7-amino-3-[(S)-tetrahydrofuran-2-yl]ceph-3-em-4-carboxylate (980mg, 38%) as a yellow foam; νₘₐₓ (CH₂Cl₂) 1777, 1721, 1613, 1516, 1152cm⁻¹; δ_{H} (CDCl₃, 250MHz) 1.53-1.71 (1H, m), 1.84-2.02 (4H, m, 2 exch.), 2.25-2.40 (1H, m), 3.31 and 3.60 (2H, ABq, J 18.5Hz), 3.78-3.98 (2H, m), 3.81 (3H, s), 4.72 (1H, d, J 5.0Hz), 4.86-4.93 (2H, m), 5.19 (2H, s), 6.88 (2H, d, J 8.6Hz), 7.33 (2H, d, J 8.6Hz) . [Mass spectrum: M⁺ (390)].

Further elution with ethyl acetate gave the more polar diastereoisomer, 4-methoxybenzyl (6R, 7R) -7-amino-3-[(R)-tetrahydrofuran-2-yl]ceph-3-em-4-carboxylate (590mg, 23%). The product was recrystallised using ethyl acetate-hexane to yield an off-white solid m.p. 131-134°C. νₘₐₓ (CH₂Cl₂) 1775, 1726, 1613, 1516, 1156cm⁻¹; δ_{H} (CDCl₃, 250MHz) 1.58-1.70 (1H, m), 1.83-2.06 (4H, m, 2 exch.), 3.38 and 3.57 (2H, ABq, J 17.8HYz), 3.77-3.93 92H, m), 3.82 (3H, s), 4.68 (1H, d, J 4.9Hz), 4.92 (1H, d, J 4.9Hz), 5.07 (1H, m), 5.22 (2H, s), 6.90 (2H, d, J 8.6Hz), 7.35 (2H, d, J 8.6Hz). [Mass spectrum: M⁺ (390)].

### Example 7

### Sodium (6R,7R)-7-[2-(2-aminothiazol-4-yl)-2-(Z)-methoxy-iminoacetamido]-3-[(S)-tetrahydrofuran-2-yl]ceph-3-em-4-carboxylate

### (a) 4-Methoxybenzyl (6R,7R)-7-[2-(2-aminothiazol-4-yl)-2-(Z)-methoxyiminoacetamido]-3-[(S)-tetrahydrofuran-2-yl] ceph-3-em-4-carboxylate

Methanesulphonyl chloride (203µl, 2.62mmol) was added to 2-(2-aminothiazol-4-yl)-2-(Z)-methoxyiminoacetic acid (528mg, 2.63mmol) and N,N-diisopropylethylamine (458µl, 2.63mmol) in DMF (8ml) at -30°C. After stirring at -30±10°C for 30min., a solution of 4-methoxybenzyl (6R,7R)-7-amino-3-[(S)-tetrahydrofuran-2-yl]ceph-3-em-4-carboxylate (930mg, 2.38mmol) in DMF (5ml) was added, followed by pyridine (213µl, 2.63mmol). The reaction mixture was transferred to an ice-bath and stirring continued for a further lh. After dilution with ethyl acetate, the solution was washed successively with saturated sodium hydrogen carbonate solution, 5% aqueous citric acid, water (x2) and brine, dried and then concentrated in vacuo. The residue was purified by chromatography on silica gel eluting with 50, 70 and 90% ethyl acetate in hexane to give the title compound as a yellow foam (1.138g, 83%); νₘₐₓ (CH₂Cl₂) 3389, 1783, 1732, 1682, 1516cm⁻¹; δ_{H} (CDCl₃, 250MHz) 1.53-1.70 (1H, m), 1.88-2.01 (2H, m), 2.28-2.41 (1H, m), 3.33 and 3.62 (2H, ABq, J 18.7Hz), 3.79-3.98 (2H, m), 3.81 (3H, s), 4.08 (3H, s), 4.94 (1H, dd, J 9.0, 6.7H , 5.04 (1H, d, J 4.8Hz), 5.18 (2H, s), 5.88 (2H, br s, xch. , 5.98 (1H, dd, J 9.0, 4.8Hz), 6.90 (2H, d, J 8.6Hz), 6.94 (1H, s), 7.35 (2H, d, J 8.6Hz), 7.50 (1H, br. d, J 9.0Hz, exch.). [Mass spectrum: +ve ion (thioglycerol) MH⁺ (574)].

### (b) Sodium (6R,7R)-7-[2-(2-aminothiazol-4-yl)-2-(Z)-methoxyiminoacetamido]-3-(S)-tetrahydrofuran-2-yl]ceph-3-em-4-carboxylate

Aluminium chloride (162mg, 1.21mmol) was added to anisole (7ml) and dry dichloromethane (3.5ml) at -20°C and stirred for 15min. The temperature of the cooling bath was then lowered to -40°C before addition of a solution of 4-methoxybenzyl (6R,7R)-7-[2-(2-aminothiazol-4-yl)-2-(Z)-methoxyiminoacetamido]-3-[(S)-tetrahydrofuran-2-yl]ceph-3-em-4-carboxylate (235mg, 0.41mmol) in dichloromethane (5ml). After 10min., the solution was treated with trisodium citrate (0.5M, 12ml) and then vigorously stirred for 10min at room temperature. The aqueous phase was separated, washed with dichloromethane (x2) and concentrated in vacuo. The residue was chromatographed on HP20SS eluting with water, then 1% THF in water.
Fractions containing the product, (h.p.l.c. analysis), were combined and freeze-dried to give the title compound (126mg, 65%); νₘₐₓ (KBr) 3401, 1761, 1669, 1603, 1533, 1040cm⁻¹; δ_{H} (d₆-DMSO, 250MHz) 1.43-1.59 (1H, m), 1.71-1.88 (2H, m), 2.0-2.12 (1H, m), 3.18 and 3.37 (2H, ABq, J 17.4Hz), 3.58 (1H, m), 3.78 (1H, m), 3.81 (3H, s), 4.87 (1H, dd, J 8.7, 6.7Hz), 4.97 (1H, d, J 4.7Hz), 5.50 (1H, dd, J 8.1, 4.7Hz), 6.74 (1H, s), 7.21 (2H, br. s, exch.), 9.48 (1H, d, J 8.1Hz, exch.). [Mass spectrum: +ve ion (thioglycerol) MH⁺ (476), MNa⁺ (498)].

### Example 8

### Pivaloyloxymethyl (6R,7R)-7-[2-(2-aminothiazol-4-yl)-2-(Z)-methoxyiminoacetamido]-3-[(S)-tetrahydrofuran-2-yl]ceph-3-em-4-carboxylate

Pivaloyloxymethyl bromide (440mg, 2.26mmol) and sodium iodide (440mg, 2.93mmol) in acetone (3ml) were stirred for 30min., filtered, and the filtrate concentrated in vacuo. The resulting iodide in toluene (2ml) was added to a solution of sodium (6R,7R)-7-[2-(2-aminothiazol-4-yl)-2-(Z)-methoxyiminoacetamido]-3-[(S)-tetrahydrofuran-2-yl]teph-3-em-4-carboxylate (560mg, 1.18mmol) in N-methylpyrrolidinone (5ml). After stirring for 45min. at room temperature, the reaction mixture was diluted with ethyl acetate, washed successively with water (x3) and brine, dried over MgSO₄ and concentrated in vacuo. The residue was chromatographed on silica gel eluting with 80% ethyl acetate in hexane to give the title compound as a yellow foam (486mg, 73%); νₘₐₓ (CH₂Cl₂) 3390, 1776, 1749, 1681, 1532cm⁻¹; δ_{H} (CDCl₃, 250MHz) 1.22 (9H, s), 1.65 (1H, m), 1.99 (2H, m), 2.41 (1H, m), 3.37 and 3.68 (2H, ABq, J 18.8Hz), 3.80-4.01 (2H, m), 4.13 (3H, s), 4.92 (1H, dd, J 8.9, 6.8Hz), 5.08 (1H, d, J 4.8Hz), 5.85 and 5.92 (2H, ABq, J 5.6Hz), 5.98 (1H, dd, J 8.4, 4.8Hz), 6.07 (2H, br s, exch.), 7.03 (1H, s), 7.40 (1H, br. d, exch. J 8.4Hz). [Mass spectrum: +ve ion (thioglycerol) MH⁺ (568)].

### Example 9

### Sodium (6R,7R)-7-[2-(2-aminothiazol-4-yl)-2-(Z)-methoxyiminoacetamido]-3-[(R)-tetrahydrofuran-2-yl]ceph-3-em-4-carboxylate

### (a) 4-Methoxybenzyl (6R,7R)-7-[2-(2-aminothiazol-4-yl)-2-(Z)-methoxyiminoacetamido]-3-[(R)-tetrahydrofuran-2-yl]-ceph-3-em-4-carboxylate

Methanesulphonyl chloride (198µl, 2.56mmol) was added to 2-(2-aminothiazol-4-yl)-2-(Z)-methoxyiminoacetic acid (515mg, 2.56mmol) and N,N-diisopropylethylamine (447µl, 2.57mmol) in DMF (8ml) at -30°C. After stirring at -30±10°C for 30min., a solution of 4-methoxybenzyl (6R,7R)-7-amino-3-[(R)-tetrahydrofuran-2-yl]ceph-3-em-4-carboxylate (915mg, 2.35mmol) in DMF (5ml) was added, followed by pyridine (207µl, 2.56mmol). The reaction mixture was transferred to an ice-bath and stirring continued for a further 1.5h. After dilution with ethyl acetate, the solution was washed successively with saturated sodium hydrogen carbonate solution, 5% aqueous citric acid, water (x2) and brine, dried and then concentrated in vacuo. The residue was triturated several times with diethyl ether to yield the title compound as an off-white solid (1.06g, 79%); νₘₐₓ (CH₂Cl₂) 3390, 1783, 1730, 1687, 1606, 1516cm⁻¹; δ_{H} (CDCl₃, 250MHz) 1.55-1.70 (1H, m), 1.86-1.98 (2H, m), 2.0-2.14 (1H, m), 3.40 and 3.59 (2H, ABq, J 17.8Hz), 3.78-3.93 (2H, m), 3.91 (3H, s), 4.12 (3H, s), 5.04 (1H, d, J 4.7Hz), 5.15 (1H, dd, J 7.7, 7.7Hz), 5.21 (2H, s), 5.87 (1H, dd, J 8.7, 4.7Hz), 6.55 (2H, br. s, exch.), 6.90 (2H, d, J 8.6Hz), 7.05 (1H, s), 7.36 (2H, d, J 8.6Hz), 7.65 (1H, br. d, J 8.7Hz). [Mass spectrum: +ve ion (thioglycerol) MH⁺ (574)].

### (b) Sodium (6R,7R)-7-[2-(2-aminothiazol-4-yl)-2-(Z)-methoxyiminoacetamido]-3-[(R)-tetrahydrofuran-2-yl]ceph-3-em-4-carboxylate

Aluminium chloride (740mg, 5.55mmol) was added to anisole (32ml) and dry dichloromethane (15ml) at -20°C and stirred for 15min. The temperature of the cooling bath was then lowered to -40°C before addition of a solution of 4-methoxybenzyl (6R,7R)-7-[2-(2-aminothiazol-4-yl)-2-(Z)-methoxyiminoacetamido]-3-[(R)-tetrahydrofuran-2-yl]ceph-3-em-4-carboxylate (1.06g, 1.85mmol) in dichloromethane (10ml). After 10min., the solution was treated with trisodium citrate (0.5M, 54ml) and then vigorously stirred for 10min. at room temperature. The aqueous phase was separated, washed with dichloromethane (x2) and concentrated in vacuo. The residue was chromatographed on HP20SS eluting with water, then 1% THF in water. Fractions containing the product, (h.p.l.c. analysis), were combined and freeze-dried to give the title compound (560mg, 64%); νₘₐₓ (KBr) 3399, 1762, 1669, 1603, 1529, 1038cm⁻¹; δ_{H} (d₆-DMSO, 250MHz) 1.50-1.91 (4H, m), 3.25 and 3.38 (2H, ABq, J 16.8Hz), 3.60-3.82 (2H, m), 3.84 (3H, s), 4.96 (1H, d, J 4.7Hz), 5.20 (1H, dd, J 8.6, 6.0Hz), 5.48 (1H, dd, J 8.1, 4.7Hz), 6.76 (1H, s), 7.23 (2H, br. s, exch.), 9.50 (1H, d, J 8.1Hz, exch.). [Mass spectrum: +ve ion (thioglycerol) MH⁺ (476), MNa⁺ (498)].

### Example 10

### Pivaloyloxymethyl (6R,7R)-7-[2-(2-aminothiazol-4-yl)-2-(Z)-methoxyiminoacetamido]-3-[(R)-tetrahydrofuran-2-yl]ceph-3-em-4-carboxylate

Pivaloyloxymethyl bromide (247mg, 1.27mmol) and sodium iodide (247mg, 1.65mmol) in acetone (5ml) were stirred for 30min., filtered, and the filtrate concentrated in vacuo. The resulting iodide in toluene (3ml) was added to a solution of sodium (6R,7R)-7-[2-(2-aminothiazol-4-yl)-2-(Z)-methoxyiminoacetamido]-3-[(R)-tetrahydrofuran-2-yl]ceph-3-em-4-carboxylate (320mg, 0.67mmol) in N-methylpyrrolidinone (5ml). After stirring for 30min. at room temperature, the reaction mixture was diluted with ethyl acetate, washed successively with water (x3) and brine, dried over MgSO₄ and concentrated in vacuo. The residue was chromatographed on silica gel eluting with 80% ethyl acetate in hexane to give the title compound as a yellow foam (297mg, 78%); νₘₐₓ (CH₂Cl₂) 3387, 1786, 1752, 1735, 1686, 1605cm⁻¹; δ_{H} (CDCl₃, 250MHz) 1.22 (9H, s), 1.69 (1H, m), 1.98 (2H, m), 2.18 (1H, m), 3.43 and 3.62 (2H, ABq, J 18.0Hz), 3.80-3.96 (2H, m), 4.10 (3H, s), 5.08 (1H, d, J 4.7Hz), 5.19 (1H, m), 5.83-5.92 (3H, m), 6.32 (2H, br. s, exch), 7.02 (1H, s), 7.63 (1H, br. d, exch., J 8.6Hz). [Mass spectrum: +ve ion (thioglycerol) MH⁺ (568)].

### Example 11

### Diphenylmethyl (6R, 7R) -7-phenylacetamido-3-[(RS)-tetrahydrofuran-2-yl]ceph-3-em-4-carboxylate

### (a) Diphenylmethyl (6R,7R)-7-phenylacetamido-3-(tetrahydrofuran-2-yl)ceph-2-em-4-carboxylate

A solution of (tetrahydrofuran-2-yl)tri-n-butylstannane (J.S. Sawyer, A. Kucerovy, T.L. MacDonald, and G.J. McGarvey, J. Amer. Chem. Soc., 1988, 110, 842) (3.0g, 8.30mmol) in THF (20ml) was cooled to -78°C. n-Butyl lithium (6.23ml of a 1.6M solution in hexane, 9.97mmol) was then added and the solution was stirred for 15min. at -78°C. A second flask containing copper (I) bromide.dimethylsulphide complex (0.854g, 4.14mmol) suspended in a mixture of dimethyl sulphide (15ml) and THF (30ml) was then cooled to -78°C. The a-lithiotetrahydrofuran species was transferred via a cannula to the suspension of copper bromide at -78°C. The red-brown homogeneous solution was stirred for 30min. at -78°C. A third flask containing a solution of diphenylmethyl 7-phenylacetamido-3-triflyloxyceph-3-em-4-carboxylate (V. Farina, S.R. Baker, and S.I. Hanck, J. Org. Chem., 1989, 54, 4962) (1.9g, 3.0mmol) in a mixture of N-methylpyrrolidinone (20ml) and THF (50ml) was then cooled to -78°C. The cuprate species was transferred via a cannula to the solution of triflate at -78°C. The reaction mixture was stirred for lh at -78°C then quenched by the addition of saturated aq. ammonium chloride (30ml). The resulting mixture was allowed to warm to room temperature then diluted with water (100ml) and extracted with ethyl acetate (100ml, 30ml). The combined organic phases were washed with water, brine, then dried over magnesium sulphate. After removal of the solvents under reduced pressure the crude reaction product was purified by flash chromatography on silica gel using 10-30% ethyl acetate/methylene dichloride as eluent. After elution of the 3-n-butylcephem, the title compound was obtained as a mixture of diastereoisomers of the Δ² and Δ³ cephems (1.014g, 61%).

### (b) Diphenylmethyl (6R,7R)-1-oxo-7-phenylacetamido-3-(tetrahydrofuran-2-yl)ceph-3-em-4-carboxylate

A mixture of the cephems (1.014g, 1.83mmol) obtained in Example 11(a) in methylene dichloride (20ml) was cooled to 0°C. A solution of m-chloroperbenzoic acid (0.52g, 60% pure, 1.81mmol) in methylene dichloride (10ml) was then added and the solution was stirred for 10min. at 0°C. The solution was washed with saturated aq. sodium hydrogen carbonate then wat and dried (MgSO₄). Evaporation of the solvent gave the title compound (1.005g, 96%) as a mixture of diastereoisomers; νₘₐₓ (KBr) 1786, 1728 and 1648cm⁻¹; δ (CDCl₃) 1.41-2.29 (4H, m), 2.99, 3.27 (together 1H, 2d, J 19Hz), 3.63 (2H, br. s), 3.63-3.87 (2.5H, m), 4.20 (0.5H, d), 4.41, 4.43 (together 1H, m), 4.97, 5.14 (together 1H, br. t, J 7.5Hz, and dd, J, 9, 6.9Hz), 6.05, 6.09 (together 1H, 2dd, J 10, 4.7Hz), 6.70, 6.82 (together 1H, 2d, J 10.1Hz), 6.87, 6.94 (together lH, 2s) and 7.26-7.40 (15H, m). [Mass spectrum: +ve ion (3-nitrobenzyl alcohol, sodium acetate) MNa⁺ (593)].

### (c) Diphenylmethyl (6R, 7R)-7-phenylacetamido-3-(tetrahydrofuran-2-yl)ceph-3-em-4-carboxylate

A solution of the sulphoxides (0.975g, 1.71mmol) obtained in Example 11(b) in DMF (20ml) was cooled to -25°C. Phosphorous trichloride (0.30ml, 3.44mmol) was then added and the solution was stirred for 10min. at -25°C. The reaction mixture was poured onto a mixture of ice, water and ethyl acetate. The organic extract was washed with water, brine, dried (MgSO₄) and evaporated. Purification by flash chromatography gave the title compound as a mixture of diastereoisomers (0.811g, 86%); νₘₐₓ (KBr) 1780, 1723 and 1663cm⁻¹; δ (CDCl₃) 1.5-2.3 (4H, m), 3.24 (0.5H, d, J 18.6Hz), 3.40 (0.5H, d, J 17.3Hz), 3.56-3.89 (5H, m), 4.84 (0.5H, dd, J 9.1, 6.7Hz), 4.95 (1H, d, J 4.8Hz), 5.01 (0.5H, br t, J 8Hz) 5.76, 5.85 (together 1H, 2dd, J 8.9, 4.8Hz), 6.01, 6.08 (together 1H, 2d, J 8.9Hz), 6.86, 6.94 (1H, 2s) and 7.26-7.38 (15H, m) [mass spectrum: M⁺ (554)].

### Example 12

### Pivaloyloxymethyl (6R,7R)-7-[2-(2-aminothiazol-4-yl)-2-(Z)-methoxyiminoacetamido]-3-(tetrahydrofuran-2-yl)ceph-3-em-4-carboxylate

### (a) Pivaloyloxymethyl (6R,7R)-7-phenylacetamido-3-(tetrahydrofuran-2-yl)ceph-3-em-4-carboxylate

A solution of the cephems (0.811g, 1.46mmol) obtained in Example 11(c) in anisole (5ml) was cooled to 0°C. Trifluoroacetic acid (10ml) was added and the mixture was stirred at 0°C for 5min. Toluene was added and the solvents were evaporated off. The residue was partitioned between water and ethyl acetate and the pH was adjusted to 7 by the addition of saturated aq. sodium hydrogen carbonate. The aqueous layer was added to ethyl acetate and the pH taken to 2 by the addition of lM HCl. The organic phase was washed with water, brine, dried (MgSO₄) and evaporated. The residue was dissolved in N-methylpyrrolidinone (3ml). Potassium carbonate (0.426g, 3.08mmol) was added followed by a solution of iodomethyl pivalate (prepared from the bromide 0.438g as in Example 2) in toluene (3ml). The mixture was stirred for 2h at room temperature, then water and ethyl acetate were added. The organic phase was washed with water, brine, dried (MgSO₄) and evaporated. The residue was purified by chromatography to give the title compound as a (5:1) mixture of diastereoisomers (0.478g, 65%); major diastereoisomer (S) δ_{H} (CDCl₃) 1.22 (9H, s), 1.56 (1H, m), 1.96 (2H, m), 2.35 (1H, m), 3.27 (1H, d, J 18.8Hz), 3.60 (1H, d), 3.65 (2H, ABq, J 16.2Hz), 3.88 (2H, m), 4.86 (1H, dd, J 9.0, 6.7Hz), 4.94 (1H, d, J 4.8Hz), 5.79-6.05 (4H, m) and 7.26-7.38 (5H, m).

### (b) Pivaloyloxymethyl (6R,7R)-7-amino-3-(tetrahydrofuran-2-yl)ceph-3-em-4-carboxylate

A solution of the diastereoisomers obtained in Example 12(a) (0.478g, 0.95mmol) in methylene dichloride (10ml) was cooled to -30°C. N-Methylmorpholine (0.206ml, 1.87mmol) was added followed by a solution of phosphorus pentachloride (0.30g, 1.44mmol) in methylene dichloride (7.5ml). The mixture was stirred at -30°C for 30min. Methanol (2.0ml) was added and the mixture was allowed to warm to room temperature over 30min. Water (2.6ml) was then added and the mixture was stirred vigorously for 1h. The mixture was concentrated by evaporation under reduced pressure and the residue was partitioned between ethyl acetate and water. The pH was adjusted to 7 with 1M aq. ammonia. The organic phase was washed with water, brine, dried (MgSO₄) and concentrated. The diastereoisomers were separated by flash chromatography to give (S)-isomer (0.195g); (Found: M⁺, 384.1363. C₁₇H₂₄N₂O₆S requires M, 384.1355); νₘₐₓ (KBr) 3408, 2977, 1780 and 1750cm⁻¹; δ (CDCl₃), 1.23 (9H, s), 1.64 (1H, m), 1.98 (2H, m), 2.10 (2H, br. s), 2.39 (1H, m), 3.35 (1H, d, J 18.7Hz), 3.63 (1H, d, J 18.6Hz), 3.90 (2H, m), 4.79 (1H, d, J 5.0Hz), 4.88 (1H, dd, J 9.1, 6.7Hz), 4.94 (1H, d, J 5.0Hz) and 5.86 (2H, m). (R)-isomer (0.046mg); δ (CDCl₃) 1.23 (9H, s), 1.6-2.4 (6H, m), 3.43 (1H, d, J 18Hz), 3.64 (1H, d, J 17.6Hz), 3.88 (2H, m), 4.79 (1H, d, J 4.9Hz), 4.99 (1H, d, J 4.9Hz), 5.17 (1H, t, J 7.5Hz) and 5.87 (2H, m).

### (c) Pivaloyloxymethyl (6R, 7R)-7-[2-(2-aminothiazol-4-yl)-2-(Z)-methoxyiminoacetamido]-3-[(S)-tetrahydrofuran-2-yl]ceph-3-em-4-carboxylate

A solution of (Z)-2-(2-aminothiazol-4-yl)-2-methoxyiminoacetic acid (0.108g, 0.537mmol) in DMF (2ml) was cooled to -50°C. N,N-Diisopropylethylamine (0.103ml, 0.59mmol) followed by methanesulphonyl chloride (0.046ml, 0.59mmol) were added and the mixture was stirred at -50°C for 30min. A further quantity of N,N-diisopropylethylamine (0.086ml, 0.493mmol) was added and this mixture was added to a precooled solution of pivaloyloxymethyl (6R,7R)-7-amino-3-[(S)-tetrahydrofuran-2-yl]ceph-3-em-4-carboxylate (0.185g, 0.482mmol) in DMF (2ml) at 0°C. The resulting mixture was stirred at 0°C for 40min., then it was partitioned between ethyl acetate and water. The organic phase was washed with water, brine, dried (MgSO₄) and evaporated. The residue was purified by flash chromatography, then triturated with ether to give the title compound (0.193g, 71%) as a white solid. The spectral data was identical with that obtained for Example 8.

### (d) Pivaloyloxymethyl (6R,7R)-7-[2-(2-aminothiazol-4-yl)-2-(Z)-methoxyiminoacetamido]-3-[(R)-tetrahydrofuran-2-yl]ceph-3-em-4-carboxylate

A solution of (Z)-2-(2-aminothiazol-4-yl)-2-methoxyiminoacetic acid (27mg, 0.134mmol) in DMF (1ml) was cooled to -50°C. N,N-Diisopropylethylamine (26µl, 0.15mmol) followed by methanesulphonyl chloride (11.5µl, 0.15mmol) were added and the mixture was stirred at -50°C for 30min. A further quantity of N,N-diisopropylethylamine (22µl, 0.126mmol) was added and this mixture was added to a precooled solution of pivaloyloxymethyl (6R,7R)-7-amino-3-[(R)]-tetrahydrofuran-2-yl]ceph-3-em-4-carboxylate (46mg, 0.12mmol) in DMF (1ml) at 0°C. The resulting mixture was stirred at 0°C for 40min., then it was partitioned between ethyl acetate and water. The organic phase was washed with water, brine, dried (MgSO₄) and evaporated. The residue was purified by flash chromatography, then triturated with ether to give the title compound (49.6mg, 73%) as a white solid. The spectral data was identical with that in Example 10.

### Example 13

### Sodium (6R,7R)-7-[2-(2-Aminothiazol-4-yl)-2-(Z)-methoxyiminoacetamido-3-[(RS)-tetrahydrofuran-3-Vl] ceph-3-em-4-carboxylate

### (a) (RS)-3-Chloroacetyltetrahydrofuran

(RS)-3-Tetrahydrofuroic acid (3.48g) in dichloromethane (40ml) was treated with oxalyl chloride (11.43g) as described in Example 1(a). The resultant acid chloride in dichloromethane (40ml) was then treated with excess diazomethane (60mM) in ether (100ml), followed by hydrogen chloride. The solution was washed once with brine, dried and concentrated. Flash chromatography on silica gel, eluting with 40% ethyl acetate/hexane afforded the title compound as a pale yellow oil, (3.924g, 88%); νₘₐₓ (CH₂Cl₂) 1735 and 1716cm⁻¹; 2.17 (2H, dt, J 7.0, 7.5Hz), 3.47-3.58 (1H, m), 3.77-4.04 (4H, m) and 4.18 (2H, s); [mass spectrum: +ve ion (ammonia) MNH₄⁺ (166)].

### (b) (3R,4R)-3-Phenylacetamido-4-[tetrahydrofuran-3-ylcarbonylmethylthio]azetidin-2-one

(RS)-3-Chloroacetyltetrahydrofuran (0.297g) was coupled with (3R, 6R)-4-mercapto-3-phenylacetamidoazetidin-2-one (0.519g) in DMF (4ml), using potassium carbonate (0.304g) as described in Example 1(b). Following work up, the crude product was taken up in hot ethyl acetate and cooled. The crystalline product was filtered off. The solvent was removed from the filtrate and the residue triturated with dichloromethane. The crystalline products were combined to give one diastereoisomer of the title compound, (0.187g, 27%); m.p. 145-155°C (decomp.); νₘₐₓ (CH₂Cl₂) 3410, 1748, 1709 (shoulder) and 1688cm⁻¹; δ_{H} ((CD₃)₂SO) 1.74-2.07 (2H, m), 3.26-3.38 (1H, m), 3.48 and 3.56 (2H, ABq, J 16.5Hz), 3.60-3.75 (4H, m), 4.87 (1H, d, J 4.5Hz), 5.24 (1H, dd, J 4.5, 8.4Hz collapses to 1H, d, J 4.5Hz with D₂O) and 9.02 (1H, d, J 8.4Hz, exchangeable with D₂O); [mass spectrum: +ve ion (3NOBA, Na+) MNa⁺ (371)]. The dichloromethane soluble residue was flash chromatographed with ethyl acetate to give the second diastereoisomer of the title compound as a colourless foam (0.162g, 23%); νₘₐₓ (CH₂Cl₂) 3407, 3302 (br), 1783 and 1681cm⁻¹; δ_{H} ((CD₃)₂SO) spectrum identical to that of previous isomer except for 3.44-3.58 (2H, m); [mass spectrum: +ve ion (3NOBA, Na⁺) MH⁺ (349), MNa⁺ (371)].

### (c) t-Butyl (RS)-2-Hydroxy-2-[(3R,4R)-3-phenylacetamido-4-[(RS)-tetrahydrofuran-3-ylcarbonylmethylthio]azetidin-2-on-1-yl]acetate

t-Butyl glyoxylate (1.601g) in 1,2-dichloroethane (20ml) was added to (3R,4R-3-phenylacetamido-4-[(RS)-tetrahydrofuran-3-ylcarbonylmethylthio]azetidin-2-one (2.712g) with triethylamine (0.079g, 0.108ml) in 1,2-dichloroethane (5ml) at room temperature; after 1h. the solution was concentrated and flash chromatographed with 70, 80 then 90% ethyl acetate/hexane to give the title compound as a colourless foam, (2.719g, 73%); νₘₐₓ (CH₂Cl₂) 3415 (br), 1780, 1735, 1685 and 1509cm⁻¹; δ_{H} (CDCl₃) 1.48 and 1.51 (9H, 2s's), 2.03-2.18 (2H, m), 3.20-3.32 (1H, m), 3.46 (1H, d, J 17.5Hz), 3.66 (2H, s), 3.69-3.97 (5H, m), 4.37 and 4.49 (1H, 2 br. d's, J 6.8 and 7.3Hz, exchangeable with D₂O), 4.98 and 5.05 (1H, 2d's, J 4.7 and 4.6Hz), 5.15-5.50 (2H, 4m's), 6.43-6.74 (1H, 3m's) and 7.32 (5H, m); [mass spectrum: +ve ion (3NOBA, Na+) MNa⁺ (501)].

### (d) t-Butyl 2-[(3R,4R)-3-Phenylacetamido-4-[(RS)-tetrahydrofuran-3-ylcarbonylmethylthio]azetidin-2-on-1-yl]-2-tri-n-butylphosphoranylideneacetate

t-Butyl (RS)-2-hydroxy-2-[(3R, 4R)-3-phenylacetamido-4-[(RS)-tetrahydrofuran-3-ylcarbonylmethylthio]azetidin-2-on-1-yl]acetate (2.719g) in THF (20ml) was treated with thionyl chloride (1.01g, 0.615ml) and 2,6-lutidine (0.913g, 0.989ml) as described in Example 1(d). Following work-up the crude chloride in dioxan (30ml) was then treated with n-butyl-phosphine (2.53g, 3.11ml). After purification by flash chromatography with 50, 70% ethyl acetate/hexane then ethyl acetate the title compound was obtained as a pale yellow foam (1.496g, 40%); νₘₐₓ (CH₂Cl₂) 3420, 1762, 1717 (shoulder), 1681 and 1625cm⁻¹. [Mass spectrum: +ve ion (3NOBA, Na⁺), MH⁺ (663), MNa⁺ (685)].

### (e) t-Butyl (6R,7R)-7-Phenylacetamido-3-[(RS)-tetrahydrofuran-3-yl]ceph-3-em-4-carboxylate

t-Butyl 2-[(3R, 4R)-3-phenylacetamido-4-[(RS)-tetrahydrofuran-3-ylcarbonylmethylthio]azetidin-2-on-1-yl]-2-tri-n-butylphosphoranylideneacetate (1.496g), thermolysed in toluene (30ml) as for Example 1(e) and purified by flash chromatography with 40, 50 and 60% ethyl acetate/hexane afforded the title compound as a yellow foam (0.28g, 28%); νₘₐₓ (CH₂Cl₂) 3411, 1702, 1718 and 1687cm⁻¹. δ_{H} (CDCl₃) 1.52 (9H, s), 1.43-2.39 (3H, m's), 3.23 and 3.44 with 3.27 and 3.44 (2H, 2ABq's J 17.7Hz), 3.51-4.03 (6H, m's), 4.94 and 4.96 (1H, 2d's, J 4.7 and 4.7Hz), 5.74-5.82 (1H, m), 6.03 and 6.04 (1H, 2d's, J 8.8 and 8.9Hz) and 7.26-7.42 (5H, m). [Mass spectrum: +ve ion (3NOBA, Na⁺) MNa⁺ (467)].

### (f) t-Butyl (6R, 7R)-7-Amino-3-[(RS)-tetrahydrofuran-3-yl]ceph-3-em-4-carboxylate

t-Butyl (6R,7R)-7-phenylacetamido-3-[(RS)-tetrahydrofuran-3-yl]ceph-3-em-4-carboxylate (0.9g) in dichloromethane (15ml) with N-methylmorpholine (0.45g, 0.49ml) was successively treated with phosphorus pentachloride (0.549g) in dichloromethane (13.74ml), methanol (10ml) and water (10ml) as described in Example 2(f). After purification by flash chromatography on silica gel eluting with 60, 80% ethyl acetate/hexane and then ethyl acetate, the title compound was obtained as a yellow solid (0.481g, 73%); (Found: M⁺, 326.1304. C₁₅H₂₂N₂O₄S requires M, 326.1300); νₘₐₓ (CH₂Cl₂) 3408, 1775 and 1716cm⁻¹; δ_{H} (CDCl₃) 1.55 (9H, s), 1.69-2.41 (3H, m's), 3.31 and 3.48 with 3.34 and 3.49 (2H, 2ABq's, J 17.5 and 17.5Hz), 3.69-3.83 (4H, 2s's overlapping m), 3.97-4.05 (2H, m), 4.72 and 4.74 (1H, 2d's, J 4.3 and 4.4Hz) and 4.95 and 4.97 (1H, 2d's, J 4.3 and 4.4Hz).

### (g) t-Butyl (6R, 7R) -7-[2-(Z) -Methoxyimino-2-(2-tritylaminothiazol-4-yl)acetamido]-3-[(RS)-tetrahydrofuran-3-yl]ceph-3-em-4-carboxylate

2-(Z)-Methoxyimino-2-(2-tritylaminothiazol-4-yl)acetic acid hydrochloride (0.751g) in DMF (5ml) was treated with methanesulphonyl chloride (0.179g, 0.121ml) and diisopropylethylamine (0.404g, 0.544ml) as described in Example 1(g). This was then treated with t-butyl (6R,7R)-7-amino-3-[(RS)-tetrahydrofuran-3-yl]ceph-3-em-4-carboxylate (0.464g) and pyridine (0.112g, 0.114ml) in DMF (5ml). Following work up and purification by flash chromatography with 40, 50 and 60% ethyl acetate/hexane, the title compound was obtained as a yellow foam (0.874g, 82%); νₘₐₓ (CH₂Cl₂) 3398, 1783, 1731 (shoulder), 1718 and 1688cm⁻¹; δ_{H} (CDCl₃) 1.53 (9H, s), 1.69-2.43 (3H, m's), 3.29 and 3.46 with 3.34 and 3.48 (2H, 2ABq's, J 17.7 and 17.7Hz), 3.63-4.07 (6H, m's and s), 5.03 and 5.06 (1H, 2d's, J 4.8 and 4.8Hz), 5.84-5.90 (1H, m), 6.73 and 6.74 (1H, 2s), 6.76 and 6.90 (1H, 2d's, J 8.7 and 8.7Hz exchangeable with D₂O), 7.02 (1H, br. s, exchangeable with D₂O) and 7.31 (15H, s). [Mass spectrum: +ve ion (3NOBA, Na⁺) MNa⁺ (774)].

### (h) Sodium (6R,7R)-7-[2-(2-Aminothiazol-4-yl)-2-(Z)-methoxyiminoacetamido-3-[(RS)-tetrahydrofuran-3-yl]ceph-3-em-4-carboxylate

t-Butyl (6R,7R)-7-[2-(Z)-methoxyimino-2-(2-tritylaminothiazol-4-yl)acetamido]-3-[(RS)-tetrahydrofuran-3-yl]ceph-3-em-4-carboxylate (0.859g) was deprotected in 10% 1M hydrochloric acid in formic acid (11.4ml) as described in Example 1(h). After work up the pH of the solution was adjusted to 8 with aqueous sodium hydrogen carbonate, and the product purified by column chromatography on HP20SS eluting with 1, 2, 4 and 6% THF/water. The fractions containing the product by h.p.l.c., were combined, concentrated and freeze-dried to give the title compound as an amorphous white solid (0.4g, 74%); νₘₐₓ (KBr) 1757, 1670, 1596 and 1532cm⁻¹; δ_{H} ((CD₃)₂SO) 1.61-2.08 (3H, m's), 3.15 and 3.37 with 3.18 and 3.37 (2H, 2ABq's, J 16.6 and 16.7Hz), 3.45-3.66 (2H, m), 3.76-3.95 (5H, m overlapping s at 3.84), 4.96 and 4.97 (1H, 2d's, J 4.3 and 4.6Hz), 5.46-5.54 (1H, m), 6.75 and 6.76 (1H, 2s's), 7.25 (2H, br s, exchangeable with D₂O). [Mass spectrum: +ve ion (thioglycerol) MH⁺ (476), MNa⁺ (498)].

### Example 14

### 4-Methoxybenzyl (6R,7R)-7-Amino-3-((R)-tetrahydrofuran-2-yl) ceph-3-em-4-carboxylate

### (a) (R)-2-Bromoacetyltetrahydrofuran

(R)-2-Tetrahydrofuroic acid (2.739g, EPA 0382 506) was converted to it's acid chloride with oxalyl chloride (9g, 6.18ml) as previously described in Example 1(a). This was dissolved in dichloromethane, cooled in ice/water and saturated with excess diazomethane, bubbled through the solution in a stream of argon. 48% Aqueous hydrogen bromide (4.41ml) was then added and the reaction mixture vigorously stirred. After 10min. the solution was washed with brine, dried and concentrated. Flash chromatography eluting with 5% then 10% ethyl acetate/hexane afforded the title compound as a pale yellow oil (3.519g, 77%); [α]_{D} + 60.9 (C 1.01 CHCl₃).

### (b) 4-Methoxybenzyl (RS)-2-Hydroxy-2-[(3R,4R)-3-phenylacetamido-4-(R)-tetrahydrofuran-2-ylcarbonylmethylthio)azetidin-2-on-1-yl]acetate

4-Methoxybenzyl (RS)-2-hydroxy-2-[(1R, 5R)-3-benzyl-4-thia-2,6-diazabicyclo[3.2.0]hept-2-en-7-on-6-yl]acetate (4.103g) in dichloromethane (15ml) and acetone (15ml) was ring opened with 4-toluenesulphonic acid hydrate (3.33g) in water (8ml) and coupled to (R)-2-bromoacetyltetrahydrofuran (2.11g) in acetone (20ml) with potassium carbonate (0.687g) as described in Example 6(b) for the diastereoisomeric mixture. After purification by flash chromatography, the title compound was obtained as a yellow gum (2.618g, 49%); [α]_{D} -10.7 (c 1.00 CHCl₃).

### (c) 4-Methoxybenzyl 2-[(3R,4R)-3-Phenylacetamido-4-[(R)-tetrahydrofuran-2-ylcarbonylmethylthio]azetidin-2-on-1-yl]-2-tri-n-butylphosphoranylideneacetate

4-Methoxybenzyl (RS)-2-hydroxy-2-[(3R,4R)-3-phenylacetamido-4-((R)-tetrahydrofuran-2-ylcarbonylmethylthio)azetidin-2-on-1-ylacetate (2.558g) was converted to the title compound with thionyl chloride (0.842g, 0.51ml), 2,6-lutidine (0.757g, 0.82ml) and tri-n-butylphosphine (2.1g, 2.58ml) as described for the diastereoisomeric mixture in Example 6(b). The product was obtained as a brown gum (2.16g, 63%).

### (d) 4-Methoxybenzyl (6R,7R)-7-Phenylacetamido-3-[(R)-tetrahydrofuran-2-yl]ceph-3-em-4-carboxylate

The phosphorane (2.16g) prepared in Example 14(c), in toluene (50ml) was heated under reflux for 8h. Removal of solvent and chromatography afforded the title compound as a yellow solid (1.008g, 67%).

### (e) 4-Methoxybenzyl (6R,7R)-7-Amino-3-((R)-tetrahydrofuran-2-yl)ceph-3-em-4-carboxylate

The cephem (0.98g), prepared in Example 14(d) was treated with phosphorus pentachloride (0.523g) in dichloromethane (13.lml) and N-methylmorpholine (0.429g, 0.466ml), then methanol (10mls) and water (10ml) as described for the diastereoisomeric mixture in Example 6(e). After work up and purification by crystallisation from toluene, the title compound was obtained as a colourless solid (0.252g, 33%); m.p. 130-132°C; [α]_{D} +11.5 (c 1.00 CHCl₃); ¹H n.m.r. was shown to be identical to that obtained for (R)-isomer prepared in Example 6(e).

### Example 15

### 4-Methoxybenzyl (6R, 7R) -7-Amino-3-((S)-tetrahydrofuran-2-yl)ceph-3-em-4-carboxylate

### (a) (S)-2-Bromoacetyltetrahydrofuran

(S)-2-Tetrahydrofuroic acid (5.94g) was converted to it's acid chloride with oxalyl chloride (13.00g). This was then converted to the title compound with diazomethane and then 48% aqueous hydrogen bromide (9.58ml) as described in Example 14(a). After isolation, the product was obtained as pale yellow oil (8.78g, 89%); [α]_{D} -62.8 (c 1.00, CHCl₃).

### (b) 4-Methoxybenzyl (RS)-2-Hydroxy-2-[(3R,4R)-3-phenylacetamido-4-((S)-tetrahydrofuran-2-ylcarbonylmethylthio)azetidin-2-on-1-yl]acetate

4-Methoxybenzyl (RS)-2-hydroxy-2-[(1R, 5R) -3-benzyl-4-thia-2,6-diazabicyc10[3.2.0]hept-2-en-7-on-6-yl]acetate (15.09g) in 50% acetone/dichloromethane (100ml) was cleaved with 4-toluenesulphonic acid (12.25g) in water (25ml). This product was reacted with the crude bromide from Example 15(a) (8.78g) in acetone (40ml) in the presence of potassium carbonate (2.53g) as described in Example 14(b). The title compound was obtained as a yellow foam (12.366g, 62%).

### (c) 4-Methoxybenzyl 2-[(3R, 4R)-3-Phenylacetamido-4-[(S)-tetrahydrofuran-2-ylcarbonylmethylthio]azetidin-2-on-1-yl]-2-tri-n-butylphosphoranylideneacetate

As for Example 14(c) the alcohol from 15(b) (12.366g) was converted to the title compound with thionyl chloride (2.47ml) and 2,6-lutidine (3.99ml) followed by tri-n-butylphosphine (12.55ml). After purification the phosphorane was obtained as a brown gum (10g, 60%).

### (d) 4-Methoxybenzyl (6R, 7R)-7-Phenylacetamido-3-[(S)-tetrahydrofuran-2-yl]ceph-3-em-4-carboxylate

As for Example 14(d) the phosphorane from Example 15(c), (10g) was cyclized in refluxing toluene (200mls). After isolation, the title compound was obtained as a yellow foam (5.452g, 78%).

### (e) 4-Methoxybenzyl (6R,7R)-7-Amino-3-((S)-tetrahydrofuran-2-yl)ceph-3-em-4-carboxylate

As for Example 14(e) the cephem from 15(d), (5.452g) was treated with phosphorus pentachloride (2.96g) and N-methylmorpholine (2.9ml) in dichloromethane (125ml), followed by treatment with methanol (50ml) then water (50ml). After adjusting the pH to 7 with 0.880 ammonium hydroxide and purification, the title compound was obtained as a pale yellow foam (2.803g, 67%); ¹H n.m.r. was shown to be identical to that obtained for the S-isomer prepared in Example 6(c).

### Example 16

### Acetoxymethyl (6R,7R)-7-[2-(2-aminothiazol-4-yl)-2-(Z)-methoxyiminoacetamido]-3-[(S)-tetrahydrofuran-2-yl]ceph-3-em-4-carboxylate

### (a) Acetoxymethyl (6R,7R)-7-phenylacetamido-3-(tetrahydrofuran-2-yl)ceph-3-em-4-carboxylate

To a solution of (6R,7R)-7-phenylacetamido-3-(tetrahydrofuran-2-yl)ceph-3-em-4-carboxylic acid (0.303g, 0.78mmol) (obtained in Example 12) in N-methylpyrrolidinone (5ml) was added potassium carbonate (0.37g, 2.66mmol). Bromomethyl acetate (0.30g, 1.95mmol) was added dropwise to the mixture over lh. The mixture was stirred for a further lh, then ethyl acetate and water were added. The organic phase was washed with water, brine, dried (MgSO₄) and evaporated. The residue was purified by chromatography to give the title compound as a mixture of diastereomers (0.198g, 56%); major diastereomer(S); δ_{H} (CDCl₃) 1.59 (1H, m), 1.95 (2H, m), 2.12 (3H, s), 2.38 (1H, m), 3.28 (1H, d, J 18.9Hz), 3.59 (1H, d), 3.65 (2H, ABq, J 16.4Hz), 3.88 (2H, m), 4.89 (1H, dd, J 9.0, 6.7Hz), 4.93 (1H, d, J 4.9Hz), 5.84 (3H, m), 6.01 (1H, d, 9.1Hz) and 7.34 (5H, m).

### (b) Acetoxymethyl (6R,7R)-7-amino-3-[(S)-tetrahydrofuran-2-yl]ceph-3-em-4-carboxylate

As for Example 12(b), the cephem from Example 16(a), (0.196g) was treated with phosphorus pentachloride (132mg) and N-methylmorpholine (94µl) in dichloromethane (7ml), followed by treatment with methanol (0.85ml) then water (1.15ml). After adjusting the pH to 7 with lM aq. ammonia and work up, the diastereomers were separated by flash chromatography to give the (S)-isomer (54.3mg, 37%); δ_{H} (CDCl₃) 1.66 (1H, m), 1.97 (2H, m), 2.13 (3H, s), 2.40 (1H, m), 3.56 (2H, ABq, J 17.6Hz), 3.91 (2H, m), 5.03 (3H, m) and 5.84 (2H, m).

### (c) Acetoxymethyl (6R,7R)-7-[(Z)-2-(2-aminothiazol-4-yl)-2-(Z)-methoxyiminoacetamido]-3-[(S)-tetrahydrofuran-2-yl]-ceph-3-em-4-carboxylate

As for Example 12(c), 2-(2-aminothiazol-4-yl)-2-(Z)-methoxyiminoacetic acid (35mg) was treated with N,N-diisopropylethylamine (34 and 27µl) and methanesulphonyl chloride (15µl) in DMF (lml)and then added to the amino compound (53mg) obtained in Example 16(b) in DMF (1ml). After work up and chromatography the title compound (60mg, 74%) was obtained as a foam; νₘₐₓ (KBr) 3330, 1774, and 1676cm⁻¹. δ_{H} (CDCl₃) 1.64 (1H, m), 1.99 (2H, m), 2.14 (3H, s), 2.41 (1H, m), 3.38 and 3.67 (2H, ABq, J 18.9Hz), 3.90 (2H, m), 4.11 (3H, s), 4.95 (1H, dd, J 9.0, 6.8Hz), 5.07 (1H, d, J 4.8Hz), 5.86 (2H, m), 5.99 (1H, dd, J 8.9, 4.8Hz), 6.08 (2H, brs), 7.00 (1H, s) and 7.49 (1H, d, J 8.8Hz). [Mass spectrum: +ve ion (ammonia) 526 (MH⁺)].

### Example 19

### Sodium (6R,7R)-7-[2-(2-Aminothiadiazol-4-yl)-2-(Z)-methoxyiminoacetamido]-3-[(S)-tetrahydrofuran-2-yl]ceph-3-em-4-carboxylate

### (a) 4-Methoxybenzyl (6R,7R)-7-[2-(Z)-Methoxyimino-2-(2-tritylaminothiadiazol-4-yl)acetamido]-3-[(S)-tetrahydrofuran-2-yl]ceph-3-em-4-carboxylate.

Mesyl chloride (65µl) was added to 2-(Z)-methoxyimino-2-(2-tritylaminothiadiazol-4-yl)acetic acid (370mg) and N,N-diisopropylethylamine (146µl) in dichloromethane (5ml) at -20°C. The reaction mixture was stirred at -20°C for 1 hour then added to an ice cold solution of 4-methoxybenzyl (6R, 7R) -7-amino-3-[(S)-tetrahydrofuran-2-yl]ceph-3-em-4-carboxylate (335mg) and pyridine (70µl) in dichloromethane (5ml). The reaction was stirred for 1 hour, concentrated and flash chromatographed on silica gel eluting with 30,50,60 and 70% ethyl acetate in hexane to afford the title compound as a foam (300mg); υ_{maX} (CHCl₃) 3398, 1784, 1724, 1691, 1516, 1134 and 1107 cm⁻¹; δ_{H} (CDCl₃, 250MHz) 1.55-1.75 (lH,m), 1.80-2.05 (2H,m), 2.25-2.45 (1H,m), 3.30 and 3.61 (2H, ABq, J 18.3Hz), 3.75-4.00 (2H,m), 3.81 (3H,m), 4.16 (3H,m), 4.85-5.00 (1H,m), 5.00 (1H,d, J 4.8Hz), 5.17 (2H,s), 5.92 (1H,dd, J 4.8Hz), 6.72 (1H,d,J 7.8Hz) and 6.88 and 7.30 (19H,m). [Mass spectrum: +ve ion (3-nitrobenzyl alcohol, sodium acetate) MNa⁺ (839)].

### (b) Sodium (6R,7R,)-7-[2-(2-Aminothiadiazol-4-yl)-2-(Z)-methoxyiminoacetamido]-3-[(S)-tetrahydrofuran-2-yl)ceph-3-em-4-carboxylate

Trifluoroacetic acid (5ml) was added to 4-methoxybenzyl (6R, 7R)-7-[2-(Z)-methoxyimino-2-(2-tritylaminothiadiazol-4-yl)acetamido]-3-[(S)-tetrahydrofuran-2-yl]ceph-3-em-4-carboxylate (100mg) and anisole (1ml) in dichloromethane (5ml) at room temperature and stirred for 1 hour. The mixture was evaporated and re-evaporated from toluene (10ml). The residue was dissolved in 1% sodium hydrogen carbonate solution (lml), washed with ether and chromatographed on HP20SS eluting with 0,0.5 and 1% acetone in water. Fractions containing the product, h.p.l.c analysis, were combined, concentrated and freeze dried to give the title compound (35mg); υₘₐₓ (KBr) 3381, 1758, 1669, 1602, 1527, 1399 and 1042cm⁻¹; δ_{H} (D₂O) 1.64-2.25 (4H,m), 3.30 and 3.49 (2H, ABq, J 17.8Hz), 3.70-3.95 (2H,m), 4.03 (3H,s), 4.65-4.75 (1H,m), 5.19 (1H,d,J 4.7Hz) and 5.77 (1H,d,J 4.7Hz). [Mass spectrum: +ve ion (thioglycerol) MH⁺(477)].

### Example 20

### (RS)-1-Acetoxyethyl (6R,7R)-7-[2-(2-Aminothiazol-4-yl)-2-(Z)-methoxyiminoacetamido]-3-[(S)-tetrahydrofuran-2-yl]ceph-3-em-4-carboxylate.

A solution of (RS)-1-acetoxyethylbromide (267mg) in 1-methyl-2-pyrrolidinone (2ml) was added dropwise, over 1 hour, to an ice cold mixture of sodium (6R,7R)-7-[2-(2-aminothiazol-4-yl)-2-(Z)-methoxyiminoacetamido]-3-[(S)-tetrahydrofuran-2-yl]ceph-3-em-4-carboxylate (190mg) and potassium carbonate (110mg) in 1-methyl-2-pyrrolidinone (1ml). After 15 minutes the mixture was diluted with ethyl acetate, washed with water, brine, dried (MgSO₄), concentrated and flash chromatographed on silica gel eluting with 50,70,80 and 90% ethyl acetate in hexane to give the title compound (172mg); υₘₐₓ (CHCl₃) 3019, 2929, 1786, 1683, 1520, 1376 and 1135cm⁻¹; δ_{H} (CDCl₃, 250MHz) 1.45-1.75 5 (4H,m), 1.90-2.10 (2H,m), 2.09 and 2.10 (together 3H, 2s), 2.30-2.50 (1H,m), 3.36 and 3.65 (2H, ABq, J 18.8Hz), 4.93-5.10 (2H,m), 5.90-6.05 (1H,m), 6.94 and 7.07 (together 1H,q,J 5.8Hz), 7.10 and 7.15 (together 1H, 2s) and 7.60 and 7.67 (together 1H,2d, J 7.4Hz); [Mass spectrum: +ve ion (3-nitrobenzyl alcohol, sodium acetate) MNa⁺ (562)].

### Example 23

### Sodium (1S, 6R, 7R)-7-[2-(2-Aminothiazol-4-yl)-2-(Z)-methoxyiminoacetamido]-3-[(S)-tetrahydrofuran-2-yl]ceph-3-em-4-carboxylate-1-oxide

### (a) 4-Methoxybenzyl (1S,6R,7R)-7-[2-(2-Aminothiazol-4-yl)-2-(Z)-methoxyiminoacetamido]-3-[(S)-tetrahydrofuran-2-yl]ceph-3-em-4-carboxylate-1-oxide

4-Methoxybenzyl (6R,7R)-7-[2-(2-aminothiazol-4-yl)-2-(Z)-methoxyiminoacetamido]-3-[(S)-tetrahydrofuran-2-yl]ceph-3-em-4-carboxylate (see example 7) (250mg, 0.44mmol) in ethyl acetate (25ml) was stirred in an ice bath and a solution of m-chloroperbenzoic acid (75mg, 0.44mmol) in ethyl acetate (5ml) was added. After 10 min the reaction mixture was washed with dilute aqueous sodium hydrogen carbonate then water followed by drying (magnesium sulphate) and evaporation under reduced pressure. The residue was chromatographed on silica gel eluting with acetone/ethyl acetate mixtures to give the title compound as a white solid (179mg, 69%); υₘₐₓ (CHCl₃) 1800, 1730, 1680 and 1610 cm⁻¹; δ_{H} (CDCl₃) 1.48-1.64 (1H,m), 1.89-2.00 (2H,m), 2.33-2.47 (1H,m), 3.29 and 3.75 (2H, ABq, J 19Hz), 3.82 (3H,s), 3.84-3.96 (2H,m), 4.09 (3H,s), 5.06 (1H,dd, J 7, 9Hz), 5.22 (2H,s), 5.55-5.8 (1H, br s, exch), 6.16 (1H,dd, J 4.5, 10Hz), 6.91 (2H,d, J 7,9Hz), 6.98 (1H,s), 7.35 (2H,d, J 9Hz) and 7.55-7.65 (1H,br, exch). [Mass spectrum: +ve ion (thioglycerol) MH⁺ (590)].

### (b) Sodium (1S, 6R, 7R)-7-[2-(2-aminothiazol-4-yl)-2-(Z)-methoxyiminoacetamido]-3-[(S)-tetrahydrofuran-2-yl]ceph-3-em-4-carboxylate-1-oxide

Anhydrous aluminium chloride (115mg, 0.86mmol) was added to a mixture of anisole (5ml) and dichloromethane (3ml) cooled to -20°C. After 15 mins at -20°C the mixture was cooled to -40°C and a solution of the product of Example 23(a) (170mg, 0.29mmol) in dichloromethane (4ml) was then added. The mixture was then stirred at -40°C for 10 min when a 0.5M aqueous solution of trisodium citrate (9ml) was added. After vigoursly stirring at room temperature the aqueous layer was separated, twice washed with dichloromethane then concentrated under reduced pressure. The residue was chromatographed on HP20SS eluting with water containing up to 2% acetonitrile. Pure fractions (as determined by HPLC)were combined and freeze dried to give the title compound as a white solid (71mg, 50%); υₘₐₓ (KBr) 1775, 1669 and 1607 (br) cm⁻¹; δ_{H} (D₂O) 1.54-].70 (1H,m), 1.94-2.03 (2H,m), 2.15-2.28 (1H,m), 3.44 and 3.85 (2H, ABq, J 18Hz), 3.8-4.0 (2H,m), 3.99 (3H,s), 4.86 (1H,t, J 8Hz), 4.99 (1H,d, J 4.5Hz), 5.95 (1H,d, J 4.5Hz) and 7.01 (1H,s). [Mass spectrum: +ve ion (thioglycerol) MH⁺(492)].

### Example 25

### Sodium (6R,7R)-7-[2-(2-Aminothiazol-4-yl)-2-(Z)-methoxy-iminoacetamidol-3-[(S)-tetrahydrofuran-2-yl]ceph-3-em-4-carboxylate-1,1-dioxide

### (a) 4-Methoxybenzyl (6R, 7R)-7-[2-(2-aminothiazol-4-yl)-2-(Z)-methoxyiminoacetamido]-3-[(S)-tetrahydrofuran-2-yl]-ceph-3-em-4-carboxylate-1,1-dioxide

To an ice-cooled solution of 4-methoxybenzyl (6R,7R)-7-[2-(2-aminothiazol-4-yl)-2-(Z)-methoxyiminoacetamido]-3-[(S)-tetrahydrofuran-2-yl]ceph-3-em-4-carboxylate (see Example 7) (300mg, 0.52mmol) in ethyl acetate (40ml) was added a solution of m-chloroperbenzoic acid (270mg, 1.56mmol) in ethyl acetate (10ml). The solution was stirred at room temperature for 1h and was then washed with dilute aqueous sodium hydrogen carbonate and water, dried (magnesium sulphate) and evaporated under reduced pressure. The residue was chromatographed on silica gel eluting with ethyl acetate/hexane mixtures to give the title compound as a cream coloured solid (50mg, 15%); νₘₐₓ (CHCl₃) 1810, 1730 and 1690cm⁻¹; δ_{H} (CDCl₃) 1.52-1.70 (1H, m), 1.94-2.00 (2H, m), 2.41-2.48 (1H, m), 3.55 and 3.85 (2H, ABq, J 19Hz), 3.19 (3H, s), 3.3-3.43 (2H, m), 4.1 (3H, s), 4.90 (1H, d, J 5Hz), 4.97 (1H, t, J 7Hz), 5.20 (2H, s), 5.94-6.3 (2H, m, exch.), 6.20 (1H, dd, J 5, 10Hz), 6.91 (2H, d, J 8Hz), 7.06 (1H, s), 7.32 (2H, d, J 8Hz) and 7.86 (1H, d, J 10Hz, exch). [Mass spectrum: +ve ion (thioglycerol) MH⁺ (606)].

### (b) Sodium (6R, 7R)-7-[2-(2-Aminothiazol-4-yl)-2-(Z)-methoxyiminoacetamido]-3-[(S)-tetrahydrofuran-2-yl]ceph-3-em-4-carboxylate-1,1-dioxide

The product from Example 25(a) was treated by the method of Example 23(b) to give the title compound (51%) as a freeze-dried white solid; νₘₐₓ (KBr) 1783, 1675 and 1610cm⁻¹; δ_{H} (d₆-DMSO) 1.45-1.50 (1H, m), 1.69-1.79 (2H, m), 2.00-2.11 (1H, m), 3.48 and 3.87 (2H, ABq, J 18Hz), 3.76 (3H, s), 3.50-3.86 (2H, m), 4.85 (1H, t, J 7Hz), 5.22 (H, d, J 5Hz), 5.61 (1H, dd, J 5, 7Hz), 6.79 (1H, s), 7.13 (2H, s, exch.) and 9.33 (lH, d, J 7Hz exch.). [Mass spectrum: +ve ion (thioglycerol) MH⁺ (508)].

### Example 26

### (RS)-1-(Propan-2-yl)oxycarbonyloxyethyl (6R,7R)-7-[2-(2-aminothiazol-4-yl)-2-(Z)-methoxyiminoacetamido]-3-[(S)tetrahydrofuran-2-yl]ceph-3-em-4-carboxylate

A solution of (RS)-1-iodo-1-(propan-2-yl)oxycarbonyloxyethane (516mg) in l-methyl-2-pyrrolidinone (2ml) was added dropwise over 45mins to an ice-cold mixture of sodium (6R,7R)-7-[2-(2-aminothiazol-4-yl)-2-(Z)-methoxyiminoacetamido]-3-[(S)-tetrahydrofuran-2-yl]ceph-3-em-4-carboxylate (237mg) and finely powdered potassium carbonate (276mg) in 1-methyl-2-pyrrolidinone (5ml). The mixture was stirred for an additional 15mins, diluted with ethyl acetate, washed with water, brine, dried (magnesium sulphate), concentrated and flash chromatographed on silica gel eluting with 50, 60, 70, 80% ethyl acetate in hexane to give the title compound as a foam (58mg); νₘₐₓ (CHCl₃) 2960, 1787, 1760, 1682, 1633, 1519 and 1377cm⁻¹; δ (CDCl₃, 250MHz) 1.20-2.50 (13H, m), 3.35-3.80 (2H, m), 3.80-4.20 (2H, m), 4.22 (3H, s), 4.83-5.10 (2H, m), 5.85-6.00 (1H, m), 6.85-7.08 (1H, m), 7.27 (1H, s) and 7.76 (1H, br, m). [Mass spectrum: +ve ion (3-nitrobenzyl alcohol, sodium acetate) MNa⁺ (606)].

### EXAMPLE 35

### 2-Ethoxycarbonyl-Z-but-2-enyl (6R,7R)-7-[2-(2-Aminothiazol-4-yl)-2-(Z)-methoxyiminoacetamido]-3-[(S)-tetrahydrofuran-2-yl]ceph-3-em-4-carboxylate

Sodium (6R,7R)-7-[2-(2-aminothiazol-4-yl)-2-(Z)-methoxyiminoacetamido]-3-[(S)-tetrahydrofuran-2-yl]ceph-3-em-4-carboxylate, (0.35g) in 1-methyl-2-pyrrolidinone (4ml) was treated with a solution of ethyl (Z)-2-bromomethylbut-2-enoate, (0.16g) in 1-methyl-2-pyrrolidinone (1ml) and stirred at ambient temperature overnight. The solution was diluted with ethyl acetate and washed with water (3x), brine and then dried. After removal of solvent in vacuo, the residue was purified by flash chromatography on silica gel, eluting with 70, 90% ethyl acetate-hexane and then ethyl acetate. The title compound was obtained as a pale yellow foam (0.368g, 86%); νₘₐₓ (CH₂Cl₂) 3480, 3389, 3320, 1781, 1726, 1682, 1606 and 1532cm⁻¹; δ_{H} (CDCl₃) 1.30 (4H, t, J 7.1Hz, overlapping M), 1.66 (1H, m), 1.97 (3H, d, J 7.3Hz), 2.35 (1H, m), 3.33 and 3.64 (2H, ABq, J 18.7Hz), 3.88 (2H, m), 4.08 (3H, s), 4.22 (2H, q, J 7.1Hz), 4.93 (1H, m), 5.05 (3H, m), 5.80 (2H, br s, exch.), 5.99 (1H, dd, J 4.8, 9.0Hz, collapses to d, J 4.8Hz on exch.), 6.83 (1H, s), 7.21 (1H, q, J 7.3Hz) and 7.73 (1H, d, J 9.0Hz, exch.). [Mass spectrum: +ve ion (thioglycerol) MH⁺ (580)].

### In Vitro Biological Data MIC (µg/ml)

| Example No. | Organism | |
|---|---|---|
| | E.coli (NCTC 1048) | S.aureus (Oxford) |
| | | |
| 1 | 0.50 | 1.00 |
| 3 | 2.00 | 1.00 |
| 7 | 0.50 | 1.00 |
| 9 | 1.00 | 0.50 |
| 13 | 1.00 | 4.00 |
| 19 | 4.00 | 2.00 |
| | | |

## Claims

1. A compound of formula (I) or a salt thereof 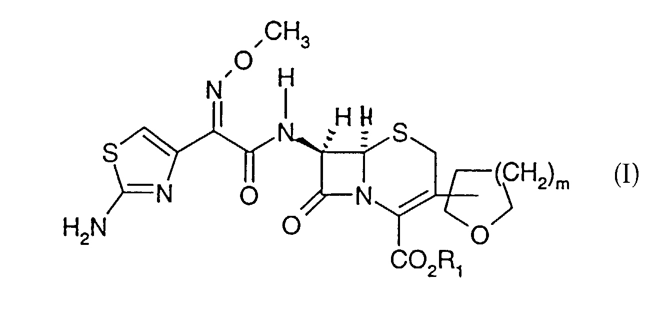 wherein m is 1 or 2,
and CO₂R₁ is a carboxy group or a carboxylate anion, or R₁ is a readily removable carboxy protecting group.

2. A compound as claimed in claim 1 having the formula (Ia) 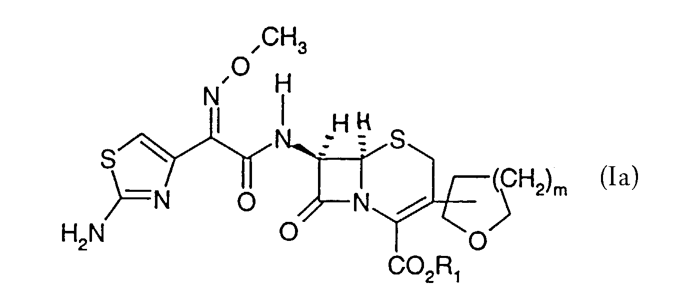 wherein the group CO₂R₁ is a carboxy group or a carboxylate anion, or a pharmaceutically acceptable salt or in vivo hydrolysable ester thereof.

3. A compound as claimed in claim 1 or 2 wherein the cyclic ether group is tetrahydrofuran-2-yl.

4. A compound selected from:
Sodium (6R,7R]-7-[2-(2-Aminothiazol-4-yl)-2-(Z)-methoxyiminoacetamido]-3-[(RS)-tetrahydrofuran-2-yl]ceph-3-em-4-carboxylate;
Pivaloyloxymethyl (6R,7R]-7-[2-(2-Aminothiazol-4-yl)-2-(Z)-methoxyiminoacetamido]-3-[(RS)-tetrahydrofuran-2-yl]ceph-3-em-4-carboxylate;
Sodium (6R,7R)-7-[2-(2-Aminothiazol-4-yl)-2-(Z)-methoxyiminoacetamido]-3-[(RS)-tetrahydropyran-2-yl]ceph-3-em-4-carboxylate;
Sodium (6R,7R)-7-[2-(2-aminothiazol-4-yl)-2-(Z)-methoxyiminoacetamido]-3-[(S)-tetrahydrofuran-2-yl]ceph-3-em-4-carboxylate;
Pivaloyloxymethyl (6R,7R)-7-[2-(2-aminothiazol-4-yl)-2-(Z)-methoxyiminoacetamido]-3-[(S)-tetrahydrofuran-2-yl]ceph-3-em-4-carboxylate;
Sodium (6R,7R)-7-[2-(2-Aminothiazol-4-yl)-2-(Z)-methoxyiminoacetamido]-3-[(R)-tetrahydrofuran-2-yl]ceph-3-em-4-carboxylate;
Pivaloyloxymethyl (6R,7R)-7-[2-(2-aminothiazol-4-yl)-2-(Z)-methoxyiminoacetamido]-3-[(R)-tetrahydrofuran-2-yl]ceph-3-em-4-carboxylate;
Sodium (6R,7R)-7-[2-(2-Aminothiazol-4-yl)-2-(Z)-methoxyiminoacetamido-3-[(RS)-tetrahydrofuran-3-yl]ceph-3-em-4-carboxylate;
Acetoxymethyl (6R,7R]-7-[2-(2-aminothiazol-4-yl)-2-(Z)-methoxyiminoacetamido]-3-[(S)-tetrahydrofuran-2-yl]-ceph-3-em-4-carboxylate;
(RS)-1-Acetoxyethyl (6R,7R]-7-[2-(2-Aminothiazol-4-yl)-2-(Z)-methoxyiminoacetamido]acetamido]-3-[(S)-tetrahydrofuran-2-yl]ceph-3-em-4-carboxylate;
(RS)-1-(Propan-2-yl)oxycarbonyloxyethyl (6R,7R)-7-[2-(2-aminothiazol-4-yl)-2-(Z)-methoxyiminoacetamido]-3-[(S)-tetrahydrofuran-2-yl]ceph-3-em-4-carboxylate; and
2-Ethoxycarbonyl-(Z)-but-2-enyl (6R,7R)-7-[2-(2-aminothiazol-4-yl)-2-(Z)-methoxyiminoacetamido]-3-[(S)-tetrahydrofuran-2-yl]ceph-3-em-4-carboxylate.

5. A compound selected from:
t-butyl (6R,7R)-7-amino-3-(tetrahydrofuran-2-yl)ceph-3-em-4-carboxylate;
4-Methoxybenzyl (6R,7R]-7-amino-3-(tetrahydrofuran-2-yl)ceph-3-em-4-carboxylate;
Pivaloyloxymethyl (6R,7R]-7-amino-3-(tetrahydrofuran-2-yl)ceph-3-em-4-carboxylate;
t-Butyl (6R,7R]-7-Amino-3-[(RS)-tetrahydrofuran-3-yl]ceph-3-em-4-carboxylate; and
Acetoxymethyl (6R,7R]-7-amino-3-[(S)-tetrahydrofuran-2-yl]ceph-3-em-4-carboxylate;

6. A pharmaceutical composition comprising a compound of Formula (Ia) as claimed in any one of claims 2 to 4 or a pharmaceutically acceptable salt or in vivo hydrolysable ester thereof, and a pharmaceutically acceptable carrier.

7. A pharmaceutical composition as claimed in claim 6 further comprising a β-lactamase inhibitor.

8. A compound of Formula (1a) or a pharmaceutically acceptable salt or in vivo hydrolysable ester thereof as claimed in any one of claims 2 to 4, for use as a medicament.

9. The use of a compound of Formula (1a) or of a pharmaceutically acceptable salt or in vivo hydrolysable ester thereof as claimed in any one of claims 2 to 4, for the manufacture of a medicament for the treatment of bacterial infections.

10. A process for the preparation of compound of formula (I) or a salt thereof as claimed in claim 1, or compound of the formula (1a) as claimed in claim 2, which comprises:
(a) treating a compound of formula (II) or a salt thereof: 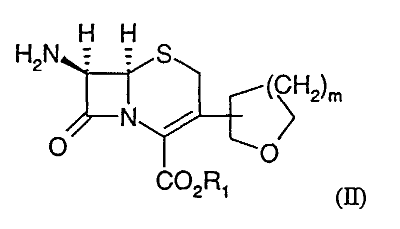 wherein CO₂R₁ and m are as defined in claim 1 or 2, and wherein any reactive group may be protected, and wherein the amino group is optionally substituted with a group which permits acylation to take place, with an N-acylating derivative of an acid of formula (III):
R²-OH (III)
where R² = 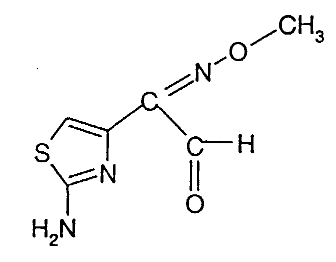 wherein any reactive group may be protected; or
(b) cyclising a compound of formula (IV): 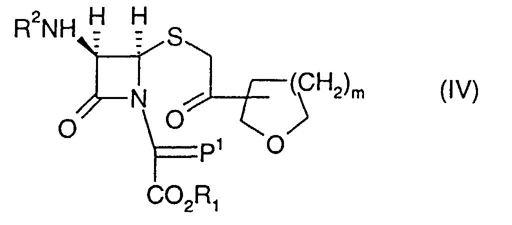 wherein R², m and CO₂R₁ are as defined in (a) above and P is a phosphorus residue; or
(c) treating a compound of formula (X): 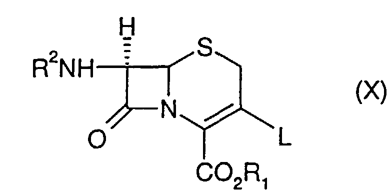 wherein R² and CO₂R₁ are as defined in (a) above and L is a leaving group, with a compound of formula (Xl): 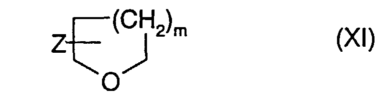 wherein Z is an organo-cuprate group and m is as defined in (a) above;
and thereafter, if necessary or desired, carrying out one of the following steps:
i) removing any protecting groups;
ii) converting the group CO₂R₁ to a different group CO₂R₁;
iii) converting the product to a salt.

11. A process according to claim 10, characterised in that a compound as claimed in any one of claims 2 to 9 is prepared.

## Patentansprüche

1. Verbindung der Formel (I) oder ein Salz davon 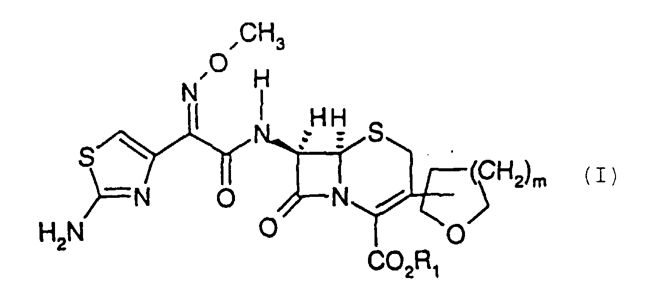 worin m 1 oder 2 ist, und CO₂R₁ eine Carboxygruppe oder ein Carboxylatanion darstellt, oder R₁ eine leicht entfernbare Carboxyschutzgruppe darstellt.

2. Verbindung nach Anspruch 1 mit der Formel (Ia) 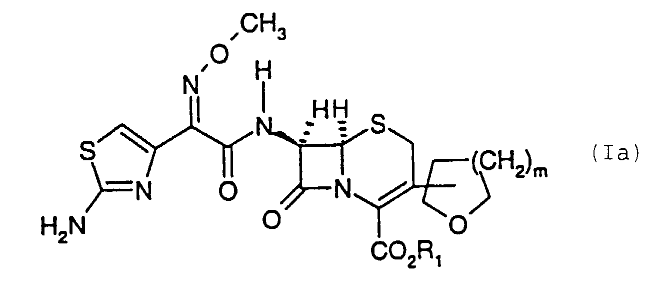 worin die Gruppe CO₂R₁ eine Carboxygruppe oder ein Carboxylatanion darstellt oder ein pharmazeutisch verträgliches Salz oder in vivo hydrolysierbarer Ester davon.

3. Verbindung nach Anspruch 1 oder 2, worin die cyclische Ethergruppe Tetrahydrofuran-2-yl darstellt.

4. Verbindung ausgewählt aus:
Natrium(6R,7R)-7-[2-(2-aminothiazol-4-yl)-2-(Z)-methoxyiminoacetamido]-3-[(RS)-tetrahydrofuran-2-yl]ceph-3-em-4-carboxylat;
(6R,7R)-7-[2-(2-Aminothiazol-4-yl)-2-(Z)-methoxyiminoacetamido]-3-[(RS)-tetrahydrofuran-2-yl]ceph-3-em-4-carbonsäurepivaloyloxymethylester;
Natrium(6R,7R)-7-[2-(2-aminothiazol-4-yl)-2-(Z)-methoxyiminoacetamido]-3-[(RS)-tetrahydropyran-2-yl]ceph-3-em-4-carboxylat;
Natrium(6R,7R)-7-[2-(2-aminothiazol-4-yl)-2-(Z)-methoxyiminoacetamido]-3-[(S)-tetrahydrofuran-2-yl]ceph-3-em-4-carboxylat;
(6R,7R)-7-[2-(2-Aminothiazol-4-yl)-2-(Z)-methoxyiminoacetamido]-3-[(S)-tetrahydrofuran-2-yl]ceph-3-em-4-carbonsäurepivaloyloxymethylester;
Natrium(6R,7R)-7-[2-(2-aminothiazol-4-yl)-2-(Z)-methoxyiminoacetamido]-3-[(R)-tetrahydrofuran-2-yl]ceph-3-em-4-carboxylat;
(6R,7R)-7-[2-(2-Aminothiazol-4-yl)-2-(Z)-methoxyiminoacetamido]-3-[(R)-tetrahydrofuran-2-yl]ceph-3-em-4-carbonsäurepivaloyloxymethylester;
Natrium(6R,7R)-7-[2-(2-aminothiazol-4-yl)-2-(Z)-methoxyiminoacetamido]-3-[(RS)-tetrahydrofuran-3-yl]ceph-3-em-4-carboxylat;
(6R,7R)-7-[2-(2-Aminothiazol-4-yl)-2-(Z)-methoxyiminoacetamido]-3-[(S)-tetrahydrofuran-2-yl]ceph-3-em-4-carbonsäureacetoxymethylester;
(6R,7R)-7-[2-(2-Aminothiazol-4-yl)-2-(Z)-methoxyiminoacetamido]acetamido]-3-[(S)-tetrahydrofuran-2-yl]ceph-3-em-4-carbonsäure-(RS)-1-acetoxyethylester;
(6R,7R)-7-[2-(2-Aminothiazol-4-yl)-2-(Z)-methoxyiminoacetamido]-3-[(S)-tetrahydrofuran-2-yl]ceph-3-em-4-carbonsäure-(RS)-1-(propan-2-yl)oxycarbonyloxyethylester und
(6R,7R)-7-[2-(2-Aminothiazol-4-yl)-2-(Z)-methoxyiminoacetamido]-3-[(S)-tetrahydrofuran-2-yl]ceph-3-em-4-carbonsäure-2-ethoxycarbonyl-(Z)-but-2-enylester.

5. Verbindung ausgewählt aus:
(6R,7R)-7-Amino-3-(tetrahydrofuran-2-yl)-ceph-3-em-4-carbonsäure-t-butylester;
(6R,7R)-7-Amino-3-(tetrahydrofuran-2-yl)-ceph-3-em-4-carbonsäure-4-methoxybenzylester;
(6R,7R)-7-Amino-3-(tetrahydrofuran-2-yl)-ceph-3-em-4-carbonsäurepivaloylmethylester;
(6R,7R)-7-Amino-3-[(RS)-tetrahydrofuran-3-yl]ceph-3-em-4-carbonsäure-t-butylester; und
(6R,7R)-7-Amino-3-[(S)-tetrahydrofuran-2-yl]ceph-3-em-4-carbonsäureacetoxymethylester.

6. Pharmazeutische Zusammensetzung, umfassend eine Verbindung der Formel (Ia) nach einem der Ansprüche 2 bis 4 oder ein pharmazeutisch verträgliches Salz davon oder einen in vivo hydrolysierbaren Ester davon und einen pharmazeutisch verträglichen Träger.

7. Pharmazeutische Zusammensetzung nach Anspruch 6, die weiterhin einen β-Lactamaseinhibitor umfaßt.

8. Verbindung der Formel (Ia) oder ein pharmazeutisch verträgliches Salz oder in vivo hydrolysierbarer Ester davon nach einem der Ansprüche 2 bis 4 zur Verwendung als Arzneimittel.

9. Verwendung einer Verbindung der Formel (Ia) oder eines pharmazeutisch verträglichen Salzes oder in vivo hydrolysierbaren Esters davon nach einem der Ansprüche 2 bis 4 zur Herstellung eines Arzneimittels zur Behandlung von bakteriellen Infektionen.

10. Verfahren zur Herstellung einer Verbindung der Formel (I) oder eines Salzes davon nach Anspruch 1 oder einer Verbindung der Formel (Ia) nach Anspruch 2, das umfaßt:
a) Behandeln einer Verbindung der Formel (II) oder eines Salzes davon: 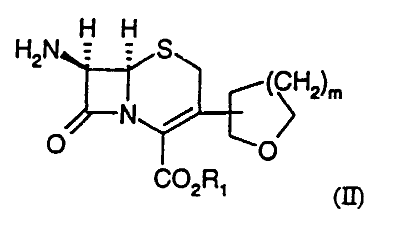 worin CO₂R₁ und m wie in Anspruch 1 oder 2 definiert sind, und worin eine beliebige reaktive Gruppe geschützt sein kann und worin die Aminogruppe gegebenenfalls mit einer Gruppe substituiert ist, die Acylierung stattfinden läßt, mit einem N-acylierenden Derivat einer Säure der Formel (III):
R²-OH (III)
worin R² = 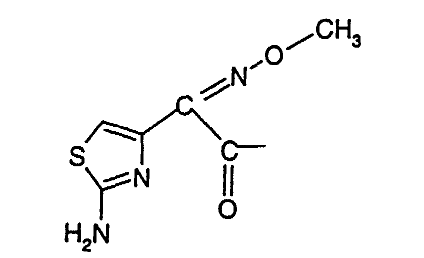 darstellt, worin jede beliebige reaktive Gruppe geschützt sein kann, oder
(b) Cyclisieren einer Verbindung der Formel (IV): 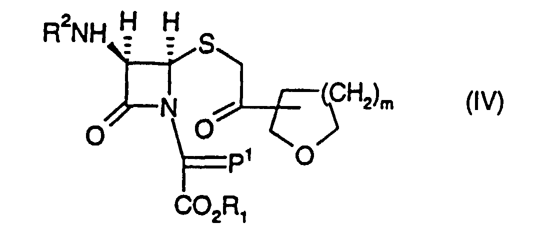 worin R², m und CO₂R₁ wie vorstehend in (a) definiert sind und P einen Phosphorrest darstellt, oder
(c) Behandeln einer Verbindung der Formel (X): 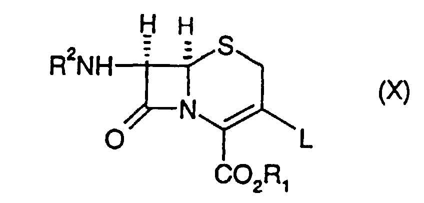 worin R² und CO₂R₁ wie vorstehend in (a) definiert sind und L eine Abgangsgruppe darstellt, mit einer Verbindung der Formel (XI), 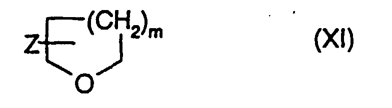 worin Z eine Organo-Cuprat-Gruppe darstellt und m wie vorstehend in (a) definiert ist;
und anschließend, falls erforderlich oder erwünscht, Ausführen eines der nachstehenden Schritte:
i) Entfernen jeglicher Schutzgruppen;
ii) Umwandeln der Gruppe CO₂R₁ zu einer anderen Gruppe CO₂R₁;
iii) Umwandeln des Produkts zu einem Salz.

11. Verfahren nach Anspruch 10, dadurch gekennzeichnet, daß eine Verbindung nach einem der Ansprüche 2 bis 9 hergestellt wird.

## Revendications

1. Composé ou sel d'un composé de formule (I) 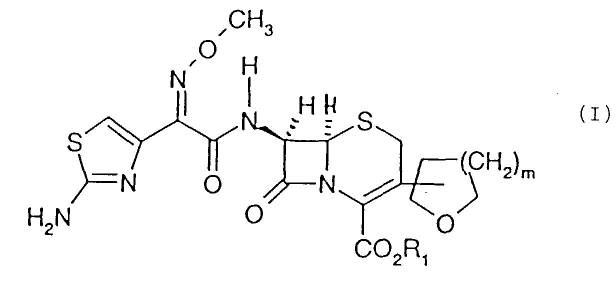 dans laquelle m a la valeur 1 ou 2,
et CO₂R₁ est un groupe carboxy ou un anion carboxylate, ou bien R₁ est un groupe aisément éliminable protégeant la fonction carboxy.

2. Composé suivant la revendication 1, répondant à la formule (la) 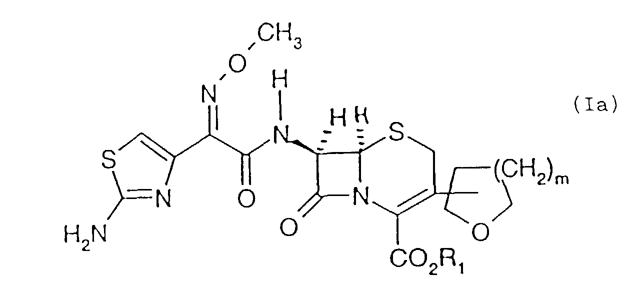 dans laquelle le groupe CO₂R₁ est un groupe carboxy ou un anion carboxylate, ou un sel pharmaceutiquement acceptable ou un ester hydrolysable in vivo de ce composé.

3. Composé suivant la revendication 1 ou 2, dans lequel le groupe éther cyclique est le groupe tétrahydrofuranne-2-yle.

4. Composé choisi entre :
le (6R,7R)-7-[2-(2-aminothiazole-4-yl)-2-(Z)-méthoxyiminoacetamido]-3-[(RS)-tétrahydrofuranne-2-yl]céph-3-ème-4-carboxylate de sodium ;
le (6R,7R)-7-[2-(2-aminothiazole-4-yl)-2-(Z)-méthoxyiminoacétamido]-3-[(RS)-tétrahydrofuranne-2-yl]céph-3-ème-4-carboxylate de pivaloyloxyméthyle ;
le (6R,7R)-7-[2-(2-aminothiazole-4-yl)-2-(Z)-méthoxyiminoacétamido]-3-[(RS)-tétrahydropyranne-2-yl]céph-3-ème-4-carboxylate de sodium ;
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate de sodium ;
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate de pivaloyloxyméthyle ;
le (6R, 7R)-7-[2-(2-aminothiazole-4-yl)-2-(Z)-méthoxyiminoacétamido]-3-[(R)-tétrahydrofuranne-2-yl]céph-3-ème-4-carboxylate de sodium ;
le (6R,7R)-7-[2-(2-aminothiazole-4-yl)-2-(Z)-méthoxyiminoacétamido]-3-[(R)-tétrahydrofuranne-2-yl]céph-3-ème-4-carboxylate de pivaloyloxyméthyle ;
le (6R,7R)-7-[2-(2-aminothiazole-4-yl)-2-(Z)-méthoxyiminoacétamido]-3-[(RS)-tétrahydrofuranne-3-yl] céph-3-ème-4-carboxylate de sodium ;
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate d'acétoxyméthyle ;
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate de (RS)-1-acétoxyéthyle ;
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate de (RS)-1-(propane-2-yl)oxycarbonyloxyéthyle ; et
le (6R,7R)-7-[2-(2-aminothiazole-4-yl)-2-(Z)-méthoxyiminoacétamido]-3-[(S)-tétrahydrofuranne-2-yl]céph-3-ème-4-carboxylate de 2-éthoxycarbonyl-(Z)-but-2-ényle.

5. Composé choisi entre :
le (6R,7R)-7-amino-3-(tétrahydrofuranne-2-yl)céph-3-ème-4-carboxylate de tertio-butyle ;
le (6R,7R)-7-amino-3-(tétrahydrofuranne-2-yl)céph-3-ème-4-carboxylate de 4-méthoxybenzyle ;
le (6R,7R)-7-amino-3-(tétrahydrofuranne-2-yl)céph-3-ème-4-carboxylate de pivaloylméthyle ;
le (6R,7R)-7-amino-3-[(RS)-tétrahydrofuranne-3-yl]céph-3-ème-4-carboxylate de tertio-butyle ; et
le (6R,7R)-7-amino-3-[(S)-tétrahydrofuranne-2-yl]céph-3-ème-4-carboxylate d'acétoxyméthyle.

6. Composition pharmaceutique comprenant un composé de formule (1a) suivant l'une quelconque des revendications 2 à 4 ou un sel pharmaceutiquement acceptable ou un ester hydrolysable in vivo de ce composé et un support acceptable du point de vue pharmaceutique.

7. Composition pharmaceutique suivant la revendication 6, comprenant en outre un inhibiteur de β-lactamase.

8. Composé de formule (1a) ou sel pharmaceutiquement acceptable ou ester hydrolysable in vivo de ce composé suivant l'une quelconque des revendications 2 à 4, destiné à être utilisé comme médicament.

9. Utilisation d'un composé de formule (1a) ou d'un sel pharmaceutiquement acceptable ou d'un ester hydrolysable in vivo de ce composé suivant l'une quelconque des revendications 2 à 4 pour la préparation d'un médicament destiné au traitement d'infections bactériennes.

10. Procédé de production d'un composé de formule (I) ou d'un sel de ce composé suivant la revendication 1, ou d'un composé de formule (la) suivant la revendication 2, qui comprend :
(a) le traitement d'un composé de formule (II) ou d'un sel de ce composé : 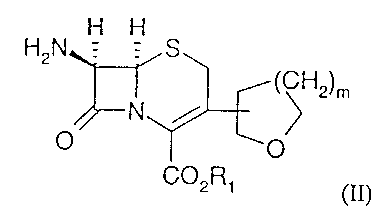 formule dans laquelle CO₂R₁ et m sont tels que définis dans la revendication 1 ou 2 et dans laquelle tout groupe réactif peut être protégé, et dans laquelle le groupe amino est facultativement substitué avec un groupe qui permet à une acylation de s'accomplir, avec un dérivé N-acylant d'un acide de formule (III) :
R²-OH (III)
dans laquelle R² est un groupe de formule 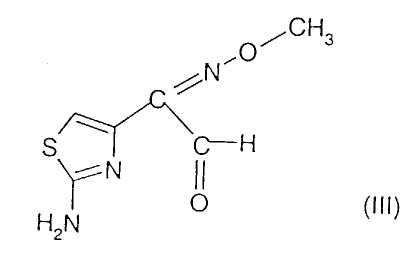 dans laquelle tout groupe réactif peut être protégé ; ou bien
(b) la cyclisation d'un composé de formule (IV): 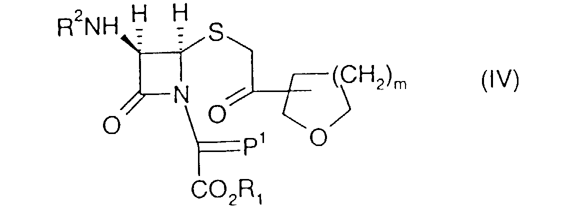 dans laquelle R², m et CO₂R₁ sont tels que définis en (a) ci-dessus et P est un résidu phosphoré ; ou bien
(c) le traitement d'un composé de formule (X) : 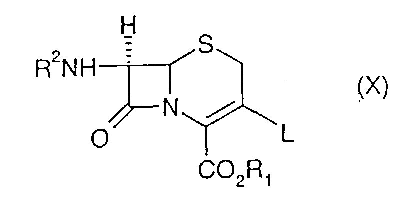 dans laquelle R² et CO₂R₁ sont tels que définis en (a) ci-dessus et L est un groupe partant, avec un composé de formule (XI) : 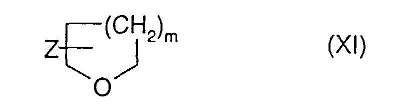 dans laquelle Z est un groupe organo-cuprate et m est tel que défini en (a) ci-dessus ;
puis, si nécessaire ou si on le souhaite, la mise en oeuvre de l'une des étapes suivantes :
i) élimination de tous groupes protecteurs ;
ii) la transformation du groupe CO₂R₁ en un groupe CO₂R₁ différent ;
iii) la transformation du produit en un sel.

11. Procédé suivant la revendication 10, caractérisé en ce qu'on prépare un composé suivant l'une quelconque des revendications 2 à 9.
